# EUROPEAN PATENT SPECIFICATION

(11) **EP 3 274 452 B1**
(45) Date of publication and mention of the grant of the patent: **03.03.2021**
(21) Application number: 16729210.1
(22) Date of filing: 27.05.2016
(51) Int. Cl.: C12N 1/06, G01N 1/28

(54) **COMPOSITION AND METHOD FOR DISRUPTING TISSUE MATERIAL**
ZUSAMMENSETZUNG UND VERFAHREN ZUR UNTERBRECHUNG VON GEWEBEMATERIAL
COMPOSITION ET PROCÉDÉ PERMETTANT D'INTERROMPRE UN MATÉRIAU TISSULAIRE

(30) Priority: 27.05.2015 EP 15169321
(43) Date of publication of application: 31.01.2018
(73) Proprietor: Qiagen GmbH, 40724 Hilden (DE)
(72) Inventor: O'NEIL, Dominic, 40724 Hilden (DE); SPERLING, Tanya, 40724 Hilden (DE); SCHROEER, Stefanie, 40724 Hilden (DE)
(74) Representative: Gille Hrabal
(86) International application number: PCT/EP2016/062002
(87) International publication number: WO 2016/189132

(56) References cited:
- WO-A1-2011/144304
- WO-A2-2005/039722
- NICK A CICCONE ET AL: "A B-cell targeting virus disrupts potentially protective genomic methylation patterns in lymphoid tissue by increasing global 5-hydroxymethylcytosine levels", VETERINARY RESEARCH, BIOMED CENTRAL LTD, LONDON, UK, vol. 45, no. 1, 23 October 2014 (2014-10-23), page 108, XP021206573, ISSN: 1297-9716, DOI: 10.1186/S13567-014-0108-5 cited in the application
- MANN P E ET AL: "Neural steroid hormone receptor gene expression in pregnant rats", MOLECULAR BRAIN RESEARCH, ELSEVIER SCIENCE BV, AMSTERDAM, NL, vol. 142, no. 1, 7 December 2005 (2005-12-07), pages 39-46, XP027784573, ISSN: 0169-328X [retrieved on 2005-12-07] cited in the application

## Description

### Introduction:

The present invention relates to a composition for disrupting tissue samples, such as in particular solid tissue, wherein the composition comprises solid disrupting particles in combination with at least one enzyme for enzymatic lysis, and at least one chaotropic agent in a total concentration equal to or less than 1M, for a combined disruption treatment by simultaneous mechanical grinding or milling disruption and enzymatic digestion. Further, the invention relates to a method for disrupting solid tissue material by simultaneously applying mechanical grinding or milling disruption and enzymatic digestion. The invention further relates to a kit and systems for carrying out solid tissue disruption in accordance with the present invention.

### Background:

The step of disruption of a sample material is one of the first and fundamental steps in analytical research, involving separating, isolating, and analyzing the desired component (analyte) from an intact sample, in particular in isolation/harvesting and analysis of cellular components such as nucleic acids, RNA, DNA, proteins, and other biochemical analytes.

In principle, both chemical and mechanical/physical methods are available for disruption of biological samples. Chemical methods are usually preferred for many sample types such as e.g., E. coli and cultured cells. Mechanical/physical methods, relying on grinding, shearing, beating and shocking are generally used for biological samples which cannot effectively be disrupted by chemical treatments, such as e.g. many microorganisms, solid tissues, solid specimens (e.g. seeds).

Known chemical disruption methods usually make use of so called lysis solutions or lysis buffers on the basis of detergents, surfactants, lytic enzymes or chaotropes, which disrupt the structure of the biological sample or cells to liberate the cellular contents or analytes.

Mechanical or physical disruption methods usually make use of homogenizers, mortar and pestles, sonicators, mixer, mills, and vortexers, which mechanically disrupt the structure of the biological sample by grinding, shearing, beating and shocking forces to liberate the cellular contents or analytes.

The choice of the disruption and homogenization method strongly depends on the kind of biological sample to be treated as well as on the cellular components to be isolated and analyzed and the choice of tools, chemistries, and their method of use may have a significant impact on the outcome of the analysis.

Solid tissue material is so far usually homogenized or disrupted by applying several steps of mechanical disruption techniques as mentioned above or by applying chemical (i.e. enzymatic) digestion over an extended period of time (usually over-night digestion). However, such methods are time consuming and need special high-performance mixing devices to achieve suitable homogenization and sufficient lysis, thereby very often deteriorating the isolated sensitive analytes. Grinding with low-power mixer such as e.g. a vortexer has been found to be not effective for grinding solid tissue material.

To use a combination of chemical disruption methods such as e.g. lysis buffers in combination with mechanical disruption methods has been mentioned very generally for example in D. W. Burden "Guide to the Disruption of Biological Samples - 2012" (Random Primers, Issue No. 12, pages 1-25, 2012), which provides a broad overview over various chemical and mechanical disruption methods for application in the disruption of various biological samples. The publication leaves open, which specific disruption methods out of the various chemical and mechanical methods may be combined or how (e.g. in subsequent treatment steps or simultaneously) for which particular biological sample type. The use of grinding balls with vortexer for rupturing tissue is mentioned, but it is pointed out, that vortexer are less suitable (less effective) for grinding tissue material due to their poor performance.

In the field of pre-treating biological samples for liberating the desired analytes, various homogenization compositions or lysis reagents are known. For example in WO 2014/096136 A2, EP 2447352 A1, or WO 1999/33559 A1 homogenization media are mentioned, which may comprise a lytic enzyme and mechanical milling particles. All of these media are described for being used in the disruption of single cells, cell cultures, microorganisms, bacteria, viruses, or spores.

International application WO 2002/00600 A1 of the present applicant relates to a method for isolating and stabilizing nucleic acids in or from micro-organisms such as prokaryotes, fungi, protozoa or algae. Therein, it is generally mentioned that compact biological samples may be homogenized or disrupted by using mechanical, chemical, physical or enzymatic methods. In Example 5 cell disruption of a bacteria sample is described and therein it is mentioned that the bacterial cell walls are disrupted using enzymatic lysis (lysozyme-mediated cell disruption) supported by the use of a bead mill with glass beads with a diameter of 150 to 600 µm at a mixing speed of 30 Hz for 5 minutes.

International application WO 2011/144304 A1 relates to a lysis buffer and a method for the lysis of bodily samples. The lysis buffer as claimed therein comprises besides at least one chaotropic agent and at least one reducing agent also at least one proteolytic enzyme. The lysis buffer may further comprise bead milling particles and the method for processing bodily samples as described therein includes bead-milling of a mixture comprising the bodily sample and the lysis buffer with the at least one proteolytic enzyme. The application specifically relates to bodily samples that are relevant for the diagnosis of respiratory diseases and therein the bodily samples are defined to comprise bodily fluids or semi-fluid samples, such as sputum, pus, secretion, aspirates, lavage, swab, or non-respiratory samples such as blood, pus, pleural fluid, pleural punctates, gastric juice, gastric aspirates, drainages or punctate fluids. The lysis buffer is mainly intended for disrupting respiratory samples and mandatorily comprises a reducing agent for dissolving mucus constituents in such samples.

EP 2 166 335 A1 of the present applicant describes a high-performance bead milling device and a method for disrupting solid tissue samples, using such device. The Example mentions that the mechanically disrupted tissue sample is subsequently treated with proteinase K and RNAse A for further analysis according to a DNeasy protocol (available from Qiagen).

WO 2005039722 A2 and the corresponding US 8,020,790 describe disruption of biological samples by using specific milling particles and a not further specified lysis buffer.

Ciccone et al. describe in their scientific publication "A B-cell targeting virus disrupts potentially protective genomic methylation patterns in lymphoid tissue by increasing global 5-hydroxymethylcytosine levels" (Veterinarys Research 2014, 45, 108) tissue disruption for a DNA blot assay using 10 mm glass beads and 10 mM tris, pH 8.0, 100 mM NaCl, 10 mM EDTA, 0.5% SDS. Therein 100 ug proteinase K are added and left to stand for protein digestion overnight at 50°C.

Mann and Babb describe in their scientific publication "Neural steroid hormone receptor gene expression in pregnant rats" (Molecular Brain Research 2005, 142, 39-46) a brain tissue sample homogenization method using a lysis buffer containing proteinase K and two 4 mm grinding beads and carrying out protein digestion at 56 °C for 30 minutes, followed by 30 minutes at -20 °C.

As shown above, a specific combination of lytic enzymes and mechanical disruption particles for a simultaneous disruption of biological samples has only been described for biological sample material, which exhibits a biological structure being characterized by comparably small size (microorganisms, cells, viruses etc.) or being structurally "weak" (single cells, cell-cultures, bodily fluids). In contrast, solid tissue material is usually characterized by a comparably stable cell construct, or fibrous or membranous structure and is much more compact as e.g. fluid or semi-fluid samples. Therefore, solid tissue material exhibits an enhanced mechanical strength or integrity, a higher density and is very often provided or sampled in the form of much larger intact / tightly connected or compact sample pieces compared to the smaller and "weaker" sample materials as described in the prior art above (e.g. cells, cell cultures, microorganisms, bacteria, viruses or spores). It is self-evident, that such solid tissue material in principal needs to be treated in a totally different way to disrupt and release the desired analytes. Disrupting the comparably tight cellular structure affords stronger forces and longer digestion times than disrupting a fluid sample or cell culture sample. Applying strong mechanical or chemical disruption forces then bears the risk of damaging or deteriorating the often very sensitive desired analytes such as e.g. nucleic acids, DNA, RNA and other cellular components.

### Object of the Invention:

It was an object of the present invention to provide a new and improved method for effectively disrupting and homogenizing human and animal derived solid tissue material for isolating and harvesting sensitive biochemical analytes, such as e.g. nucleic acids, RNA, DNA and other cellular components, which avoids the disadvantages of the prior art methods. Preferably, the new and improved method for disrupting solid tissue should be particularly mild. More preferably, the new method should provide a mild disruption and homogenization of solid tissue samples, improved quality of the isolated analytes, work faster and simplify the sample preparation in particular for automated analyses with high throughput. Even more preferably, the new and improved method should be carried out by applying mild mechanical and/or mild chemical impact on the tissue sample, in particular by applying reduced mechanical forces and/or reduced chemical impact. Even more preferred the chemical impact of chaotropic agents should be reduced. Further, it is preferred that the activity of the lytic enzyme is improved and its inactivation is avoided to provide its optimal efficacy in a combined mechanical and chemical (i.e. enzymatic) lysis treatment of the tissue sample. It is further preferred to provide a faster method with shortened disruption / digestion times.

It was surprisingly found, that with the compositions, kits, systems and the method according to the present invention this object has been solved. The compositions, kits, systems and the method of the present invention allow effective disruption of human and animal derived solid tissue material, even of very tough tissue samples with highly compact or tight tissue structure such as e.g. rodent tails (e.g. mouse tails), in a significantly shorter time period, even when carried out with usual lab equipment such as low-power vortexer, and simultaneously achieves higher yields of the desired analytes with better quality of the analytes compared to the so far applied homogenization methods for intact tissue material.

Surprisingly it turned out, that the present invention in particular improves the extraction of DNA out of tissue samples compared to either enzymatic digestion or mechanical disruption with bead mills alone. Chemical, i.e. enzymatic digestion alone is generally unable to maximize yields extracted out of tissue samples. Mechanical bead milling disruption alone usually requires specialized and large equipment (bead mills) in order to efficiently homogenize tissue samples for efficient DNA extraction. In the present invention it has been shown that the combination of an optimized lysis chemistry in combination with an optimized choice of grinding particles allows efficient, fast, and complete disruption and homogenization of tissue samples, even on common desktop vortexers, thereby massively improving the yield compared to using chemical, i.e. enzymatic digestion alone, and in addition, surprisingly the quality (especially the size) of the extracted nucleic acid was not deteriorated by the milling procedure and turned out to be superior to that as achieved by usual bead milling treatments.

It has in particular surprisingly been found that with the specific combination of the mechanical milling conditions and the specific chemical lysis conditions of the present invention a more than additive effect in the sense of a kind of synergistic effect can be achieved, compared to applying either the mechanical milling conditions or the specific chemical lysis conditions alone.

### Description of the Invention:

The subject matter of the present invention are new compositions and methods for the disruption and homogenization of human and animal derived solid tissue material.

In the context of the present invention the term "tissue material" or "tissue" relates to human and animal derived solid tissue samples, such as in particular whole or intact tissue samples. The term "tissue material" or "tissue" includes cellular organizational level intermediates between cells and a complete organ as well as samples of human or animal bodies comprising such cellular organizational level intermediates. The solid tissue material in the sense of the present invention is usually characterized by a comparably stable cell construct or cell organization, or a fibrous or membranous structure. Usually such tissue material is tougher and much more compact or tightly connected as e.g. simple cell cultures or fluid or semi-fluid samples. Accordingly, solid tissue material in the sense of the present invention exhibits an enhanced mechanical strength or integrity, a higher density and is very often sampled in the form of much larger intact / tightly connected or compact sample pieces compared to small and "weak" sample materials as described in the prior art above (e.g. single cells, cell cultures, microorganisms, bacteria, viruses or spores). In particular tissue material according to the present invention comprises, without being limited, connective tissue, muscle tissue, nervous tissue, epithelial tissue and mineralized tissue. Accordingly, tissue in the sense of the present invention includes - without being limited - organized (connected) cell constructs, fibrous and/or membranous tissue, comprising for example skin, muscle, tendons, filaments, nerves, cartilage, bone, organs, such as for example intestine, gastric, liver, spleen, brain, lymph, bone marrow, kidney, heart, as well as tail (such as rodent tail, e.g. mouse tail) etc. Preferably, the tissue material as used in the present invention is the result of a biopsy. More preferably, the tissue material as used in the present invention is solid tissue compared to fluid or semi-fluid samples such as e.g. blood, mucous membrane. Further, it is preferred that the tissue material as used in the present invention is tough solid tissue compared to weak single cells or cell cultures.

In the context of the present invention the term "disruption" or "disrupting" comprises all levels of disruption of the sampled and treated tissue material, which allows liberation or release of cellular components or the desired analytes from the tissue sample in an amount above the individual detection limit of the respective analyte or in an amount which allows isolation, harvesting and detection with suitable analysis techniques. Therein, a high degree of disruption includes complete or at least partial homogenization of the tissue sample. Homogenization means that the tissue sample is brought to a state such that all (or at least the homogenized parts) of the fractions of the sample are essentially equal in composition. This means that a homogenized sample is disintegrated, milled or minced and then mixed so well that removing some of the sample does not alter the overall molecular make-up of the remaining sample, and is identical to the removed fraction. Preferably the composition, kit and systems as well as the method of the present invention relates to disruption and/or homogenization of human and animal derived solid tissue material.

The invention relates to the use of a composition as defined in claims 1 to 6 for disrupting human and animal derived solid tissue material.

Further, the invention relates to a composition for disrupting human and animal derived solid tissue material as defined in claims 7 and 8.

Further, the invention relates to a kit for disrupting human and animal derived solid tissue material as defined in claim 9.

The composition for the disruption of tissue material according to the present invention comprises
- one or more solid disrupting particles;
- at least one enzyme for enzymatic lysis;
- at least one chaotropic agent in a total concentration equal to or less than 1M; and preferably
- at least one buffer.

Preferably the composition for the disruption of human and animal derived solid tissue material according to the present invention comprises
- one or more solid disrupting particles;
- at least one enzyme for enzymatic lysis;
- at least one chaotropic agent in a total concentration equal to or less than 1M; and
- at least one buffer.

More preferably the composition for the disruption of human and animal derived solid tissue material according to the present invention comprises
- one or more solid disrupting particles;
- at least one enzyme for enzymatic lysis;
- at least one buffer;
- at least one chaotropic agent in a total concentration below or equal to 1 M; and
- at least one anti-foaming agent.

In a further preferred embodiment, the composition for the disruption of human and animal derived solid tissue material according to the present invention further comprises one or more agents selected from
- at least one chaotropic agent;
- at least one detergent;
- at least one nuclease inhibitor;
- at least one anti-foaming agent;
- at least one osmotic stabilizer;
- at least one reducing agent,
and any mixture thereof.

Further preferred embodiments relate to the compositions for the disruption of tissue material as defined above, comprising one or more solid disrupting particles, at least one enzyme for enzymatic lysis, at least one buffer, at least one chaotropic agent in a total concentration of chaotropic agent below or equal to 1 M, and at least one anti-foaming agent and one or more agents selected from
- at least one detergent;
- at least one nuclease inhibitor;
- at least one reducing agent;
and any mixture thereof.

It is particularly preferred that the compositions for the disruption of tissue material as defined above do not comprise an organic solvent, such as in particular alcohols, in concentrations which effect denaturation and thus inactivation of the at least one lytic enzyme. For example ethanol, propanol and/or iso-propanol should not be present in the composition of the present invention in an amount which denatures or inactivates the enzymes (such as in particular the lytic enzymes as defined below, specifically proteases such as particularly proteinase K). Most preferably, the compositions of the present invention do not comprise organic solvents, in particular no alcohols, very particularly no methanol, ethanol, propanol and/or iso-propanol.

Solid disrupting particles in the context of the present invention comprise solid particles, preferably in the form of solid beads, spheres, balls, cones, cylinders, cubes, triangles, rectangles and similar suitable geometric forms as well as irregular shapes, for example so-called ballcones and satellites (shaped like Saturn, , planet or UFO), for effecting a disruption of the tissue material when mixing or milling forces are applied to the tissue sample in the composition of the present invention. However, the solid disrupting particles should be selected in view of not deteriorating or disrupting the released cellular components or analytes.

To achieve sufficient disruption and homogenization of the tissue material and undamaged liberation of the desired analytes for further isolation, harvesting and analysis it is preferred that the solid disrupting particles of the present invention are solid inert particles, i.e. particles made of a material, which does not react with the tissue material, with any of the reagents of the composition and in any case not with the desired analytes to be liberated upon disruption. It is particularly preferred that the isolated analytes are not adsorbed by or do not adhere to the inert solid disrupting particles. Suitable inert materials comprise for example inert metals, steel, stainless steel, metal oxides, glass (silica), plastic, and ceramic. Examples of inert materials comprise ZrO₂, SiO₂, Al₂O₃, Fe₂O₃, TiO₂, zirconium silicate, metals and alloys from tantalum, platinum, etc.. Further suitable inert disrupting materials are known from commercially available inert disrupting particles. It is also possible to use mixtures of one or more kind of disrupting particles, i.e. use disrupting particles of different forms and/or made of different inert materials.

It is further preferred that the disrupting particles exhibit a sufficient hardness so that no abrasion occurs during the milling or grinding process.

Preferred are beads, spheres or balls (so called milling beads), preferably stainless steel beads, ceramic beads, such as in particular zirconium beads. More preferred are irregularly shaped irregularly shaped milling particles such as ballcones or satellites, in particular made of steel or stainless steel.

To achieve sufficient disruption forces to effectively disrupt and in particular homogenize the treated tissue sample the disrupting particles should preferably exhibit a comparably large size - compared to the beads as used in the prior art for disrupting microorganisms or cell cultures, which have a size of 100 µm to approximately 600 µm. Increasing the amount of small sized beads (100 µm to about 600 or 800 µm beads) for increasing the disruption forces is not a suitable approach, as the high amount of such small particles damages or even destroys the sensitive analytes such as in particular DNA.

It is therefore preferred that the disrupting particles according to the present invention exhibit a size of at least 1 mm, preferably more than 1 mm. More preferably the particles exhibit a size of at least 1.5 mm, more preferably of more than 2 mm (> 2 mm), preferably more than 2.5 mm. Further, the disrupting particles may preferably exhibit a size of at least 3 mm (≥ 3 mm), more preferably of at least 4 mm, more preferred of at least 5 mm.

It is further preferred that the disrupting particles according to the present invention exhibit a size of up to 15 mm, preferably up to 12 mm, more preferably up to 11.5 mm.

The disrupting particles according to the present invention may exhibit a size of 1 mm or more than 1 mm to 15 mm, 1.5 mm to 15 mm, more than 2 mm to 15 mm, 2.5 mm to 15 mm, 3 mm or more than 3 mm to 15 mm, 4 mm to 15 mm. The particles may further exhibit a size of 1 mm or more than 1 mm to 12 mm, 1.5 mm to 12 mm, more than 2 mm to 12 mm, 2.5 mm to 12 mm, 3 mm or more than 3 mm to 12 mm, 4 mm to 12 mm. Further, the disrupting particles may exhibit a size of 1 mm or more than 1 mm to 11.5 mm, 1.5 mm to 11.5 mm, more than 2 mm to 11.5 mm, 2.5 mm to 11.5 mm, 3 mm or more than 3 mm to 11.5 mm, 4 mm to 11.5 mm. The particles may further exhibit a size of 1 mm or more than 1 mm to 7 mm, 1.5 mm to 7 mm, more than 2 mm to 7 mm, 2.5 mm to 7 mm, 3 mm or more than 3 mm to 7 mm, 4 mm to 7 mm. Most preferred is a size of 3 mm or more than 3 mm to 7 mm or of 4 mm to 7 mm.

The defined sizes of the disrupting particles indicate the longest distance between two opposite points of the respective particle. Accordingly, in the case of round or essentially round particles (beads, balls, spheres), the defined size relates to the diameter. In the case of irregularly shaped particles such as satellites or ballcones the longest distance between two opposite points is usually the diameter of the "saturn-like ring" surrounding the ball or ballcone part of such particles.

Depending on the size of the disrupting particles, one or more disrupting particles can be used. In the case of very large particles, the desired results of disruption and preservation of the analytes may be achieved with only one particle (in particular one ball or ballcone). It is preferred to use one disrupting particle.

Suitable disrupting particles comprise 1.0 to 1.7 mm beads, 2.8 to 3.0 mm beads, 7/64" grinding balls (approximately 2.8 mm) (in particular stainless steel grinding balls), 5/32" grinding balls (approximately 6.0-7.0 mm), 6 mm particles (in particular zirconium satellites), 3/8" grinding balls (approximately 9.5 mm) and 7/16" grinding balls (approximately 11.1 mm). Preferred are 5/32" beads, and essentially round 5 mm particles (e.g. beads, balls, spheres). Further examples of commercially available particles of irregular shape, ballcones or satellite-shaped particles, which are also preferred, exhibit the following sizes:

| **Sizes [mm]** | | | |
|---|---|---|---|
| **ball diameter x ring diameter** | **ball diameter (A)** | **ring diameter (B)** | **height (top of the cone to the opposite located side of the ball (C)** |
| 3 x 5 mm | 3 mm | 5 mm | 3.6 mm |
| 4 x 6 mm | 4 mm | 6 mm | 4.7 mm |
| 5 x 7 mm | 5 mm | 7 mm | 5.7 mm |
| 6.5 x 8.5 mm | 6.5 mm | 8.5 mm | 8 mm |

Therein, one half of the steel ball-cone is a semi-sphere (ball (A)), the other half is a cone and both are separated by a sloping central flange (ring (B)).

It is also possible to use mixtures of disrupting particles of different sizes. Further it is possible to mix disrupting particles of any form, material and size, as defined herein.

The enzyme for enzymatic lysis according to the present invention is selected from the group of hydrolases (according to the group EC3 in the EC number classification of enzymes). In particular, the at least one enzyme for enzymatic lysis is selected from the group of proteases (EC 3.4), which are often also designated as peptidases, proteinases, or proteolytic enzymes. Preferably, the at least one enzyme for enzymatic lysis is selected from the group of proteinaseK, collagenase, dispase, trypsin, pepsin, most preferred is proteinaseK.

Besides the at least one enzyme for enzymatic lysis (proteolytic enzyme) at least one additional enzyme, other than the enzyme for enzymatic lysis, may be added. Preferably, such additional enzyme is selected from the group of hydrolases acting on ester bonds according to enzyme class EC 3.1. Preferably as such an additional enzyme an RNase may be added, preferably RNase A is added.

The at least one buffer according to the present invention may be any buffer, which is suitable for receiving the tissue sample, the at least one enzyme for enzymatic lysis (the proteolytic enzyme(s)) and the one or more optional additional agents in the composition of the present invention. In particular, the buffer must be compatible with the enzyme for enzymatic lysis and must not completely inhibit the activity of the proteolytic enzyme or of any additional agent of the composition. Preferably, a buffer may be chosen, which stabilizes the isolated analytes such as e.g. isolated nucleic acids, DNA, RNA or other cellular components. Suitable buffers comprise TRIS buffer, PBS buffer, Good's buffers, SSC, sodium citrate, sodium acetate, phosphate buffers, and biological buffers, selected from the list comprising for example MES, Bis-Tris, ADA, ACES, PIPES, MOPSO, Bis-Tris Propane, BES, MOPS, TES, HEPES, DIPSO, MOBS, TAPSO, Trizma, HEPPSOPOPSO, TEA, EPPS, Tricine, Gly-Gly, Bicine, HEPBS, TAPS, AMPD, TABS, AMPSO, CHES, CAPSO, AMP, CAPS, CABS. Preferred are buffers having a pH ≥ 6 (equal to or above 6). If the desired analyte to be liberated from the tissue material is DNA, then a buffer having a pH ≥ 7 (equal to or above 7) is preferred. If the desired analyte to be liberated from the tissue material is RNA, then a buffer having a pH ≥ 6 (equal to or above 6) is preferred. Particularly preferred are the buffers from the list of biological buffers above. More preferably the at least one buffer is selected from TRIS buffer, PBS buffer.

Chaotropic agents (or chaotropes) are effective to support or increase chemical digestion, may help to reduce nuclease activity and help to denature proteins, which can cause havoc on freshly homogenized samples. In the use according to the present invention, in principle all common chaotropes may be used, such as for example sodium iodide, guanidine hydrochloride (guanidine HCl, GuHCl), guanidinium thiocyanate (GTC), guanidine isothiocyanate, and urea. Guanidinium hydrochloride is preferred in the present invention. The addition of a chaotrope is particularly suitable, when the disrupted tissue material is intended for subsequent nucleic acid isolation procedures, in particular procedures which use silica based resins/gels for purification of the isolated nucleic acid analytes. Further, chaotropes are particularly suitable, when the desired released analyte is RNA.

Generally, chaotropes are used at comparably high molarities. For example guanidine salts are usually used at 6 M concentrations, in particular for RNA isolation. Sodium iodide is also generally used at 6 M. Urea is often used at 9.5 M. The inventors of the present invention surprisingly found that the new compositions, kits and systems and the method of the present invention allows a significantly reduced concentration of chaotropic agents, so that the concentration of the at least one chaotropic agent (total concentration of chaotropic agents) is equal to or less than 1 M (≤1 M). In particular it is preferred to use chaotropes in a (total) concentration of from 0.5 M to 1 M. The use of the reduced concentrations of chaotropic agent of not more than 1 M is advantageous as chaotropes such as GuHCl and GTC are toxic and a reduction thereof is thus desired for safety reasons and to protect the users of such compositions. Further, high concentrations of chaotropes will decrease the effectiveness of proteinaseK, due to their protein denaturing effects. Accordingly, reducing the concentration of chaotropes, to not more than 1M, effects a reduced chemical impact on the tissue sample and allows to provide a more gentle or mild lysis composition.

Surfactants (often also designated as detergents) may support the lysis of the tissue sample and support solubilization of the homogenate. The addition of at least one surfactant is particularly preferred for disruption of fatty tissue such as liver or brain. Surfactants comprise ionic surfactants such as anionic and cationic surfactants, zwitterionic surfactants and non-ionic surfactants.

The group of anionic surfactants comprises for example sodium dodecyl sulfate (SDS), sodium deoxycholate, sodium lauryl ether sulfate (SLES, sodium laureth sulfate), and sodium myreth sulfate, with SDS being preferred.

The group of cationic surfactants comprises for example cetyltrimethylammonium bromide (CTAB), cetyl trimethylammonium chloride (CTAC), cetylpyridinium chloride (CPC), benzalkonium chloride (BAC), benzethonium chloride (BZT), 5-bromo-5-nitro-1,3-dioxane, dimethyldioctadecylammonium chloride, cetrimonium bromide, dioctadecyldimethylammonium bromide (DODAB).

The group of non-ionic surfactants comprises for example Triton X-100, Tween 20 (polysorbate 20, polyoxyethylene (20) sorbitan monolaurate), Brij-35 (polyalkylenglycolether), NP-40 (nonyl phenoxypolyethoxyl ethanol), Nonidet P-40 (octylphenoxypolyethoxyethanol), with Triton X-100 and Tween 20 being preferred.

The group of zwitterionic surfactants comprises for example phospholipids phosphatidylserine, phosphatidylethanolamine, phosphatidylcholine, and sphingomyelins.

Preferably the at least one surfactant is selected from the group of non-ionic surfactants as defined above. More preferably, the at least one surfactant is selected from the group consisting of SDS, Tween20 and Triton X-100. Even more preferred the at least one surfactant is selected from Tween20 and Triton X-100, and it is very particularly preferred to use a combination of both, Tween20 and Triton X-100.

Nuclease inhibitors according to the present invention comprise for example EDTA, PMSF, pepstatin A, leupeptin, aprotinin. Preferably the at least one nuclease inhibitor is EDTA.

Further, it is possible to add at least one anti-foaming agent (or defoamer), which may reduce and prevent the formation of foam in the reaction mixture and thus improve the further processability. Anti-foaming agents may be used to prevent formation of foam or may be added to break an already formed foam. In principle, all commonly used anti-foaming agents can be used, such as for example insoluble oils, polydimethylsiloxanes and other silicones, certain alcohols, stearates and glycols - provided that they do not negatively affect any of the reaction agents, the tissue sample, the isolated analytes or disturb any subsequent isolation, harvesting and analysis methods. A preferred anti-foaming agent is polydimethylsiloxane.

Osmotic stabilizers may be added to help bind up water and prevent dissociation of related solutes. Osmotic stabilizers comprise for example sucrose or sorbitol.

As osmotic stabilizers may interfere with the cell lysis, the presence of an osmotic stabilizer is less preferred, and it is even more preferred not to add osmotic stabilizers and thus further improve tissue disruption and homogenization with the compositions of the present invention.

According to the present invention it is also possible to add at least one reducing agent. Reducing agents may particularly be added, when the desired analyte to be isolated from the disrupted tissue is RNA, as the reducing agent reduces RNase. Reducing agents comprise for example glutathione, dithiothreitol, and β-mercaptoethanol
Very particularly it is preferred that the compositions of the present invention comprise a reducing agent if the desired analyte is RNA and that in such case the composition does not comprise a RNase.

However, as reducing agents may cause undesired side-reactions and are uncomfortable in the working process (e.g. due to off odors) it is preferred not to add reducing agents to the compositions, kits and systems or in the method of the present invention. In particular when the desired analyte to be isolated from the disrupted tissue is DNA the addition of a reducing agent is avoided or preferably even excluded.

Therefore, a preferred embodiment of the present invention relates to a composition according to the present invention, which consists of
- one or more solid disrupting particles;
- at least one enzyme for enzymatic lysis;
- at least one buffer, preferably selected from TRIS and PBS buffer;
- at least one chaotropic agent in a total concentration equal to or less than 1M, preferably guanidinium hydrochloride;
   and optionally one or more agents selected from
- at least one surfactant, preferably selected from the group of non-ionic surfactants, preferably selected from Tween20 and Triton X-100;
- at least one nuclease inhibitor, preferably EDTA;
- at least one anti-foaming agent, preferably polydimethylsiloxane;
- at least one osmotic stabilizer, preferably selected from sucrose and sorbitol; and
- at least one further enzyme selected from the group of RNases, preferably RNase.

A further preferred embodiment of the present invention relates to a composition according to the present invention, which consists of
- one or more solid disrupting particles;
- at least one enzyme for enzymatic lysis;
- at least one buffer, preferably selected from TRIS and PBS buffer;
- at least one chaotropic agent in a total concentration equal to or less than 1M, preferably guanidinium hydrochloride;
- at least one surfactant, preferably selected from the group of non-ionic surfactants, preferably selected from Tween20 and Triton X-100;
- at least one nuclease inhibitor, preferably EDTA; and
- at least one further enzyme selected from the group of RNases, preferably RNase.

A further preferred embodiment of the present invention relates to a composition according to the present invention, which consists of
- one or more solid disrupting particles;
- at least one enzyme for enzymatic lysis;
- at least one buffer, preferably selected from TRIS and PBS buffer;
- at least one chaotropic agent in a total concentration equal to or less than 1M, preferably guanidinium hydrochloride;
- at least one surfactant, preferably selected from the group of non-ionic surfactants, preferably selected from Tween20 and Triton X-100; and
- at least one nuclease inhibitor, preferably EDTA.

A further preferred embodiment of the present invention relates to a composition according to the present invention, which consists of
- one or more solid disrupting particles;
- at least one enzyme for enzymatic lysis;
- at least one buffer, preferably selected from TRIS and PBS buffer;
- at least one chaotropic agent in a total concentration equal to or less than 1M, preferably guanidinium hydrochloride;
- at least one surfactant, preferably selected from the group of non-ionic surfactants, preferably selected from Tween20 and Triton X-100; and
- at least one further enzyme selected from the group of RNases, preferably RNase.

A further preferred embodiment of the present invention relates to a composition according to the present invention, which consists of
- one or more solid disrupting particles;
- at least one enzyme for enzymatic lysis;
- at least one buffer, preferably selected from TRIS and PBS buffer;
- at least one chaotropic agent in a total concentration equal to or less than 1M, preferably guanidinium hydrochloride; and
- at least one surfactant, preferably selected from the group of non-ionic surfactants, preferably selected from Tween20 and Triton X-100.

The present invention further relates to a kit for disrupting human and animal derived solid tissue material, the kit comprising
- one or more solid disrupting particles;
- at least one enzyme for enzymatic lysis;
- at least one buffer, preferably selected from TRIS and PBS buffer;
- guanidinium hydrochloride in a concentration equal to or less than 1 M;
   and optionally one or more agents selected from
- at least one surfactant, preferably selected from the group of non-ionic surfactants, preferably selected from Tween20 and Triton X-100;
- at least one nuclease inhibitor, preferably EDTA;
- at least one anti-foaming agent, preferably polydimethylsiloxane;
- at least one osmotic stabilizer, preferably selected from sucrose and sorbitol;
- at least one reducing agent, preferably selected from glutathione, dithiothreitol and beta-mercaptoethanol;
- at least one additional enzyme, other than the enzyme for enzymatic lysis, preferably RNase;
   and
- optionally a container for receiving the tissue material; and further
- at least one organic solvent, preferably selected from alcohols, preferably ethanol and/or isopropanol;
- and a leaflet with instructions for processing the tissue material.

It is particularly preferred that the kit comprises a reducing agent if the desired analyte is RNA and in such case it is further preferred that the kit does not comprise a RNase.

The container for receiving the tissue material may be any suitable container or reaction vessel, which is inert with respect to the agents used in the disruption treatment, which exhibits enough mechanical stability to withstand the milling or grinding forces of the disrupting particles without being destroyed or abraded, which exhibits a suitable size for receiving the sample material, the lysis solution and the one or more selected disrupting particle and still provides suitable space to allow agitation and movement of the inserted components to effect tissue disruption, and which can suitably be used with the device which is used for effecting the milling or grinding of the human and animal derived solid tissue material by the disrupting particles. Suitable container or reaction vessels (tubes) are known and commonly available.

The addition of an organic solvent such as in particular of at least one alcohol to the readily disrupted and/or homogenized tissue sample is advantageous for providing excellent binding conditions of the liberated analytes onto silica surfaces in subsequent purification, isolation and harvesting steps, as described below in more detail. Suitable alcohols to be included in a kit comprise - without being limited - methanol, ethanol, propanol, iso-propanol, etc. Preferably ethanol and/or iso-propanol is added. As explained above, organic solvents effect denaturation and thus potentially inhibit the at least one lytic enzyme of the composition for disrupting the tissue sample. Accordingly, the organic solvents in such kits are separated from the compounds for the tissue disruption and are added after the tissue disruption has been carried out.

Further embodiments of a kit according to the present invention comprise a combination of components (reagents) corresponding to the compositions as defined above for the tissue disruption and optionally a container for receiving the tissue material and at least one organic solvent, preferably selected from alcohols, preferably ethanol and/or isopropanol and a leaflet with instructions for processing the human and animal derived solid tissue material.

The kit according to the present invention may further comprise suitable components for carrying out the above mentioned subsequent binding, purification, isolation and harvesting steps. It is particularly preferred to provide a combination of the above mentioned kit for effecting tissue disruption according to the invention in combination with a binding buffer comprising the chaotropic agent and iso-propanol. However, it is also possible to use and provide the above mentioned kit for effecting tissue disruption according to the invention in combination with known and commercially available test kits for carrying out DNA or RNA binding, as well as with commercially available test kits for carrying out DNA or RNA purification and analysis. Accordingly, the above mentioned kits may further comprise compounds corresponding to available test kits such as for example DNeasy or RNeasy kits (available from Qiagen) or the respective test kits themselves and/or materials for absorption and purification such as suitable silica based membranes, columns (for example QIAamp columns available from Qiagen) or silica magnetic beads.

The present invention further relates to a system (a combination, kit-of-parts) comprising a composition or a kit as defined above and a device for effecting the mechanical milling or grinding of the human and animal derived solid tissue material by the disrupting particles.

Such device for effecting the mechanical milling or grinding may be any common device used in grinding or bead milling techniques, comprising for example high-power mixing mills, high-performance mixer or mills, high-speed mixer, common bead mills as well as low-power mixers, such as common laboratory vortexer, bench-top vortexer, or common lab shaker (e.g. horizontal shaker).

As mentioned above, from the prior art it was already known to use milling beads and bead mills for disrupting intact human and animal derived solid tissue material. However, for achieving efficient homogenization of the human and animal derived solid tissue material generally specialized and large milling equipment such as bead mills or high-power / high-performance mixer had to be used. The present invention now surprisingly provided a possibility for disrupting or homogenizing tissue material efficiently, fast and completely, even with low-power mixers such as common desktop vortexers, which belong to the standard equipment of nearly every laboratory. Accordingly, as a device for effecting the milling or grinding of the tissue low-power mixer, including in particular vortexer or horizontal shaker, are preferred. Particularly preferred are vortexer.

High-power or high-performance mixer or mills usually work with a frequency of 15 to 60 Hz.

Low-power mixer such as in particular common vortexer usually work with a force of 150 up to 3200 rpm.

Applying a reduced mechanical power, e.g. from a low-power mixer or vortexer is advantageous for preserving the quality of the released analytes and avoid damages or deterioration of the analytes due to massive mechanical impact.

A further object of the present invention relates to a system (a combination, kit-of-parts) comprising a composition, a kit or the system as defined above, and further comprising a human and animal derived solid tissue material, which is intended for disruption by the composition, kit or system of the present invention. Regarding the human and animal derived solid tissue material of such system, reference is made to the above definition of "tissue material".

The present invention further relates to a new method for disrupting or homogenizing tissue material, wherein the tissue material is subjected to a simultaneous treatment of
- mechanical disruption by grinding, milling or beating and
- chemical disruption by enzymatic lysis,
by grinding, milling or beating the tissue material in a lysis solution which comprises one or more solid disrupting particles, at least one enzyme for enzymatic lysis, and the chaotropic agent in a total concentration equal to or less than 1M.

The method according to the present invention is characterized in that the grinding, milling or beating of the tissue material is effected by one or more solid disrupting particles as defined above. With respect to preferred embodiments and selections, reference is made to the embodiments and selections as defined above.

The method according to the present invention is preferably characterized in that the disruption of the tissue material is carried out or effected by using a device for effecting the milling or grinding of the tissue as defined above, such as preferably high-power mixing mills, high-speed mixers, or low-power mixers including vortexers, in particular by using low-power mixers or vortexers as defined above.

The method according to the present invention is preferably carried out by using any of the compositions, kits or systems (combinations, kit-of-parts) as defined above.

It is particularly preferred, that when carrying out the method of the present invention organic solvents, such as in particular alcohols are not present in the compositions for the disruption of human and animal derived solid tissue material in concentrations which effect denaturation and thus inactivation of the at least one lytic enzyme. As defined above, for example ethanol, propanol and/or iso-propanol should not be present in an amount which denatures or inactivates the enzymes. Most preferably, no organic solvents, in particular no alcohols, very particularly no ethanol, propanol and/or iso-propanol are present when carrying out the method of disrupting the human and animal derived solid tissue material.

The method according to the present invention is in particular characterized by comprising the steps:
(i) adding to a tissue material in a suitable container (e.g. as defined above in context with the kit)
   a) one or more solid disrupting particles, the at least one buffer, the at least one enzyme for enzymatic lysis, the at least one chaotropic agent in a total concentration equal to or less than 1M, optionally an RNase, and optionally one or more agents selected from, surfactants, reducing agents, nuclease inhibitors, anti-foaming agents or mixtures thereof, or
   b) any of the composition as defined above;
(ii) optionally closing the container;
(iii) disrupting the tissue material in the container using a high-power mixing mill, a high-speed mixer, or a low-power mixer, including a vortexer, preferably by using a vortexer;
(iv) incubating the mixture of step (iv) at a temperature above 24 °C, preferably between 25 and 70 °C;
(v) optionally repeating step (iii) and (iv);
(vi) optionally providing the resulting mixture for subsequent purification, separation, extraction and/or analytic process steps; or alternatively storing the resulting mixture, preferably after deactivation of the lytic reagents, in particular the lytic enzymes, e.g. by addition of a suitable reagent for deactivation and/or by cooling or freezing of the resulting mixture.

The preferred milling time according to step (iii) is about 1 minute, preferably about 30 seconds to about 15 minutes, preferably about 30 seconds to about 5 minutes. When a high-power or high-performance mixer or mill is used, the milling time is preferably about 30 seconds. When a low-power mixer such as in particular a vortexer is used, the milling time is preferably about 5 minutes.

The lysis time according to step (iv) does preferably not exceed 4 hours, more preferably 3 hours, even more preferably 2 hours, much more preferably 1 hour and even more preferred less than 1 hour such as particularly less than 30 minutes. If step (iv) is repeated (step (v)), then the total lysis time should not exceed 4 hours, preferably 3 hours, more preferably 2 hours, much more preferably 1 hour and even more preferred less than 1 hour such as particularly less than 30 minutes. Preferably, the lysis time according to step (iv) (or the total lysis time respectively) is at least 5 minutes, more preferably at least 10 minutes. Preferably with the method of the present invention a sufficient tissue disruption can be achieved to be completed in 15 minutes.

In particular, when the method is used for DNA extraction, the defined lysis times are preferred.

It is in particular preferred that the tissue material is not in contact with the lysis solution of the present invention for more than 4, preferably 3, more preferably 2 hours, much more preferably more than 1 hour and even more preferred more than 30 minutes. Very particularly an over-night digestion (at least 8 hours digestion) shall be avoided. In particular, when the method is used for DNA extraction.

With the compositions, kits, systems (combinations, kit-of-parts) and method of the present invention the disruption and homogenization time can be significantly reduced down to 1 or 2 hours and even more to 30 minutes or even less, such as particularly to not more than about 15 minutes. In contrast, common techniques need a minimum average digestion time of at least 150 minutes (2.5 hours), while over-night digestion (at least 8 hours digestion) is usual.

The present invention is particularly suitable for extracting nucleic acids, DNA, RNA, miRNA (microRNA), mRNA, tRNA, rRNA, etc. from human and animal derived solid tissue material as defined above.

It is particularly preferred to use the present invention for extracting DNA, RNA and miRNA out of tissue samples, extraction of DNA is very particularly preferred.

Thus, the method of the present invention may comprise one or more additional steps, subsequent to step (vi) above, comprising
- collection of the processed material;
- stabilization of the processed material; e.g. by inactivating the lytic agents, in particular the lytic enzymes, e.g. by freezing the mixture;
- storing the processed (and stabilized) material;
- binding of the analytes, e.g. by adding additional chaotrope and/or organic solvents such as alcohols, in particular ethanol and/or iso-propanol;
- purification of the analytes, e.g. using silica based membranes, columns or silica magnetic bead based techniques;
- isolation of the analytes, e.g. using elution techniques;
- concentration of the analytes;
- collection of the analytes;
- stabilization of the purified / isolated analytes;
- storing of the purified / isolated analytes;
- analyzing, e.g. PCR-analysis.

It is in particular possible to use the processed tissue material resulting from step (vi) above as the starting material in known and commercially available purification, isolation and analysis tests or to use commercially available test kits for the further processing.

The present invention further relates to the use of the compositions, the kits and the systems (combinations, kit-of-parts) as defined herein for treating human and animal derived solid tissue material as defined above. Therein treating preferably relates to disruption and/or homogenization as defined above, but may also comprise releasing or adding the tissue sample to a composition or mixture according to the present invention.

Without limiting the scope of the present invention, the following examples shall illustrate the present invention:

### Examples:

Extraction of DNA from rat tissue samples

### Tissue Material:

rat, stabilized with RNALater (available from Qiagen)
Liver 25 mg, muscle 25 mg, lung 10 mg, kidney 20 mg, heart 10 mg

### Equipment:

Container for receiving the tissue sample
Tube: 2ml screw cap tube (PP, Sarstedt - skirted base)

### Disrupting particles

Bead: 5/32" Ballcone (ABBOTTBall),
round 5 mm stainless steel beads,
faceted zirconium beads

### Device for effecting the milling / grinding

A) Vortex Genie 2 (Scientific Industries SI-V524), with various adapters: foam insert, horizontal and vertical Microtube Holder (VortexD)
B) TissueLyser II (available from Qiagen)
C) TissueLyser LT (available from Qiagen)

### Lysis:

20 µl proteinaseK
4 µl RNaseA
200 µl AVE buffer and
40 µl VXL buffer (available from Qiagen)

### Binding and Washing:

265 µl MVL buffer,
500 µl AW1 buffer,
500 µl AW2 buffer,
50 - 100 µl ATE buffer (available from Qiagen))

### Notes to the chemical agents

- ProteinaseK is very active in this concentration of chaotropic salt, but likely any concentration from 1M down to 0.5M Guanidine may be used.
- Tween20 and TritonX-100 are present as surfactants (detergents), though other surfactants would also be effective. Surfactants are not necessary for all tissues, but are not harmful to the process for any tissue, and improve the results when using particularly fatty tissue such as liver or brain.
- The chemistry is not restricted to column-based procedures, Silica magnetic-bead-based procedures are equally effective.

### Protocol:

1. Excising the tissue sample or removing it from storage
2. Weighing and cutting the tissue sample into a suitably sized slice (5-25 mg), then placing the piece into a 2 ml screw cap tube (Sarstedt 72.608+ blue cap 65.716.001), adding one or more of the disrupting particles mentioned above, e.g. one 5/32" Ballcone (ABBOTTBall), or several of the round 5 mm beads or of the faceted zirconium beads
3. Adding the lysis reagents and enzymes as listed above and closing the lid.
   200µl AVE / 40µl VXL / 1µl DX Reagent / 20µl Proteinase K / 4µl RNase A (100mg/ml) and closing the lid.
   For processing multiple samples a master Digestion Buffer Mix can be prepared.
4. Proceeding with the homogenization:
   A) Vortex Genie2 with appropriate 2 ml tube adapter: 5 minutes at full speed (3200 rpm)
   B) TissueLyser II with 2 x 24 2ml microcentrifuge adapter: 30 seconds at 24Hz
   C) TissueLyser LT: 1-2 minutes at 30-45Hz
5. Incubating at 56°C and 1200 rpm in a Thermoshaker for 10 minutes
   Note: Incubation proceeded after disruption regardless of visible residual tissue.
   If after incubation there was still residual tissue left, homogenization of the tissue was carried out a second time:
   A) Vortex Genie2 with Vertical Microtube Holder: 3 minutes at full speed (3200 rpm)
   B) TissueLyser II: 15 seconds at 20Hz
   C) TissueLyser LT: 45 seconds at 30-40Hz
   Then, the sample was incubated again at 56 °C and 1200 rpm in a Thermoshaker for 10 more minutes.
6. Opening the tube and adding 265 µl MVL buffer (available from Qiagen), mixing by pipetting or vortexing
7. Applying the mixture from step 6 to a QIAamp Mini Spin Column (available from Qiagen), closing the cap, followed by centrifugation for 1 minute at full speed (not exceeding 20000 x g), placing the Spin Column in a new 2 ml collection tube and discarding the collection tube containing the filtrate
8. Opening the Spin Column and adding 500 µl AW1 buffer (available from Qiagen), closing the cap, followed by centrifugation for 30 seconds at full speed (not exceeding 20000 x g), placing the Spin Column in a new 2 ml collection tube and discarding the collection tube containing the filtrate
9. Opening the Spin Column and adding 500 µl AW2 buffer (available from Qiagen), closing the cap, followed by centrifugation for 30 seconds at full speed (not exceeding 20000 x g), placing the Spin Column in a new 2 ml collection tube and discarding the collection tube containing the filtrate
10. Centrifugation at full speed for 2 minutes (to eliminate the chance of possible AW2 carryover), placing the Spin Column in a clean 1.5 ml microcentrifuge tube
11. Opening the Spin Column and adding 100 µl ATE buffer (available from Qiagen), incubation at room temperature for 1 minute, followed by centrifugation at full speed (not exceeding 20000 x g) for 1minute
12. Repeating step 11.

### A. Determination of yield across multiple sample types compared to classic ProteinaseK digestion

### Determination of the yield

UV-VIS-spectrophotometer (Nanodrop) / DNA-specific fluorescent assay (Qubit 2.0); 0,6 %TAE gel:
18 h 20 volt,
template: same volume of eluate
QFPathogenmitlCDNA-Assay with 12000 copies ICDNA/reaction and 1 µl/6µl eluate addition.

### Test Results:

**Table 1**

| **Prep** | **Disruption** / **Lysis Buffer Mix** | **+Buffer** | **Disruption Device/Particle** | **Mechanical Disruption** | | **Chemical Disruption /Incubation** | | **Binding** | **DNA Yield [µg], MV n=2** | | |
|---|---|---|---|---|---|---|---|---|---|---|---|
| | | | | **I/II** | **III** | **I/II** | **III** | | **I Lung 11mg** | **Liver 25mg** | **III Muscle 25mg** |
| QIAamp Fast | 40µl VXL / 20µl PK / 4µl RNaseA / 1µl DX | w/o Azid 200µl PBS | Vortex (D) + 5/32" ballcone | 5min full speed | 8min full speed | 56°C / 1000rpm / 15min | 56°C / 1000rpm / 20min | 265µl MVL | 53,55 | 39,504 | 10,9872 |
| | | 200µl PBS w/Azid | | | | | | | 36,306 | 37,824 | 5,8458 |
| | | 200µl H₂O | | | | | | | 30,324 | 46,176 | 8,9544 |
| | 180µl ATL / 20µl PK | - | | | | | | 200µl AL / 200µl 96-100% EtOH | 23,904 | 30,558 | 5,1756 |
| | 40µl VXL / 20µl PK / 4µl RNaseA / 1µl DX | 200µl PBS w/o Azid | TissueLyser II +5/32" ballcone | 1min 24Hz | 1min 24Hz | | 56°C/ 1000rpm / 15min | | 49,44 | 39,17 | 8,43 |
| | | 200µl PBS w/Azid | | | | | | 265µl MVL | 38,23 | 44,61 | 8,21 |
| | | 200µl H2O | | | | | | | 52,89 | 47,62 | 8,31 |
| | 180µl ATL / 20µl PK | - | | | | | | 200µl AL / 200µl 96-100% EtOH | 30,04 | 46,45 | 7,93 |
| QIAamp Mini | 180µl ATL +20µl PK | - | - | - | | 56°C / 1000rpm / 120min | | 200µl AL / 200µl 96-100% EtOH | 6,33 | 30,88 | 1,27 |

| | | | | | | | | | | | |
|---|---|---|---|---|---|---|---|---|---|---|---|
| Material: Rat tissue, RNALater stabilized; Vortex (D): VortexGenie2 +Vertical Microtube Holder(SI-V524) | | | | | | | | | | | |

**Table 2**

| **Prep** | **Disruption/Lysis Buffer Mix** | **+Buffer** | **Disruption Device /Particle** | **mechanical Disruption** | | **Chemical Disruption/Incubation** | | **Binding** | **DNA Yield [µg], MV n=2** | | |
|---|---|---|---|---|---|---|---|---|---|---|---|
| | | | | **I/III** | **II** | **I/III** | **II** | | **I Lung 14mg** | **II Muscle 25m** | **III Kidney 20mg** |
| QIAamp Fast | 40µl VXL / 20µl PK / 4µl RNaseA / 1µl DX | 200µl PBS w/o Azid | Vortex (D) + 5/32" ballcone | 5min full speed | 8min full speed | 56°C / 1000rpm / 15min | 56°C / 1000rpm / 20min | 265µl MVL | 36 | 8,586 | 50,28 |
| | | 200µl PBS w/Azid | | | | | | | 45,18 | 9,132 | 53,58 |
| | | 200µl H2O | | | | | | | 31,8 | 9,672 | 45,54 |
| | 40µl VXL / 20µl PK / 4µl RNaseA / 1µl DX | 200µl PBS w/o Azid | TissueLyser II +5/32" ballcone | 1min 24Hz | 1min 24Hz | 56°C / 1000rpm / 15min | 56°C / 1000rpm / 15min | 265µl MVL | 30,54 | 6,37 | 58,98 |
| | | 200µl PBS w/Azid | | | | | | | 31,74 | 7,19 | 67,20 |
| | | 200µl H₂O | | | | | | | 29,88 | 6,74 | 67,20 |
| QIAamp Mini | 180µl ATL +20µl PK | - | - | - | | 56°C / 10 150 | 00rpm / min | 200µl AL / 200µl 96-100% EtOH | 1,51 | 0,10 | 0,99 |

| | | | | | | | | | | | |
|---|---|---|---|---|---|---|---|---|---|---|---|
| Material: Rat tissue, RNALater stabilized; Vortex (D): VortexGenie2 +Vertical Microtube Holder(SI-V524) | | | | | | | | | | | |

**Table 3**

| **Prep** | **Dsiruption/Lysis Buffer Mix** | **+Buffer** | **Disruption Device/Particle** | **Mechanical Disruption** | | **Chemical Disruption/Incubation** | | **Binding** | **DNA Yield [µg], MV n=2** | | |
|---|---|---|---|---|---|---|---|---|---|---|---|
| | | | | **I** | **II/III** | **I** | **II/III** | | **I Muscle 25mg** | **II Liver 25mg** | **III Lung 11mg** |
| QIAamp Fast | 40µl VXL / 20µl PK / 4µl RNaseA / 1µl DX | 200µl PBS w/o Azid | Vortex (D) + 5/32" ballcone | 8min full speed | 5min full speed | 56°C / 1000rpm /20min | 56°C / 1000rpm / 10min | 265µl | | | |
| | | | | | | | | | 12,78 | 45,18 | 48,42 |
| | | 200µl PBS w/Azid | | | | | | | 10,95 | 44,04 | 23,34 |
| | | 200µl H2O | | | | | | | 10,368 | 46,32 | 32,4 |
| | | 200µl AVE | | | | | | | 8,19 | 48,06 | 35,04 |
| | 40µl VXL/ 20µl PK / 4µl RNaseA / 1µl DX | 200µl PBS w/o Azid | TissueLyser II +5/32" ballcone | 1min 24Hz | 1min 24Hz | 56°C / 1000rpm / 10min | | | MVL 9,37 | 72,60 | 49,32 |
| | | 200 µl PBS w/Azid | | | | | | | 7,27 | 55,62 | 56,76 |
| | | 200µl H₂O | | | | | | | 7,28 | 70,80 | 52,86 |
| | | 200ml AVE | | | | | | | 5,34 | 71,34 | 66,60 |
| QIAamp Mini | 180µl ATL + 20µl PK | | - | | | 56°C/ 1000rpm / 150min | | 200µl AL / 200µl 96-100% EtOH | 0,00 | 0,47 | |

| | | | | | | | | | | | |
|---|---|---|---|---|---|---|---|---|---|---|---|
| Material: Rat tissue, RNALater stabilized; Vortex (D): VortexGenie2 +Vertical Microtube Holder(SI-V524) | | | | | | | | | | | |

**Table 4**

| **Prep** | **Dsiruption/Lysis Buffer Mix** | **+Buffer** | **Disruption Device/Particle** | **Mechanical Disruption** | | **Chemical/Incubation Disruption** | | **Binding** | **DNA Yield [µg], MV n=2** | |
|---|---|---|---|---|---|---|---|---|---|---|
| | | | | **I** | II | **I** | **II** | | **Muscle 25mg** | II **Heart 10mg** |
| QIAamp Fast | 40µl VXL / 20µl PK / 4µl RNaseA / 1µl DX | 200µl PBS w/o Azid | Vortex (D) + 5/32" ballcone | 8min full speed | 5min full speed | 56°C / 1000rpm / 20min | 56°C / 1000rpm / 10min | 265µl MVL | 12,462 | 15,96 |
| | | 200µl PBS w/Azid | | | | | | | 8,988 | 13,02 |
| | | 200µl H₂O | | | | | | | 8,568 | 13,956 |
| | | 200µl AVE | | | | | | | 11,982 | 14,76 |
| | 40µl VXL/ 20µl PK / 4µl RNaseA / 1µl DX | 200µl PBS w/o Azid | TissueLyser II +5/32"ballcone | 1min 24Hz | 1min 24Hz | 56°C / 1000rpm / 10min | | | 14,52 | 15,06 |
| | | 200µl PBS w/Azid | | | | | | | 13,68 | 19,56 |
| | | 200µl H₂O | | | | | | | 11,13 | 17,94 |
| | | 200ml AVE | | | | | | | 10,49 | 16,02 |
| QIAamp Mini | 180µl ATL + 20µl PK | - | - | - | 56°C / 1000rpm / 180min | | | 200µl AL / 200µl 96-100% EtOH | 0,08 | 0,99 |

| | | | | | | | | | | |
|---|---|---|---|---|---|---|---|---|---|---|
| Material: Rat tissue, RNALater stabilized; Vortex (D): VortexGenie2 +Vertical Microtube Holder(SI-V524) | | | | | | | | | | |

Yields of nucleic acid from tissue types were up to 20-fold improved over a standard proteinaseK digestion. Overall digestion time was also reduced by a factor of five compared to proteinaseK digestion alone (in both cases, digestion was carried out until the sample appeared visibly homogenous).

Average proteinaseK digestion time (without mechanical milling) was 150 minutes, while the new method achieved homogenization in generally less than 30 minutes.

The experiment also shows that equivalent yields can be achieved on a common laboratory vortexter (low-power device) and in an expensive high-power bead mill.

Further, it has been shown that also DNA quality (DNA size) was improved through the use of vortexer.

### B. Experimental time effort compared to classic ProteinaseK digestion

The effectiveness of the method of the present invention has been evaluated in view of the time effort compared to classic proteinaseK digestion, applying the test conditions as given above.

### Test Results

**Table 5**

| **Prep** | **Disruption /Lysis Buffer Mix** | **Disruption Device/Particle** | **Mechanical Disruption** | | **Chemical Disruption/Incubation** | | | **Binding** | **DNA Yield [µg], MV n=2** | | | |
|---|---|---|---|---|---|---|---|---|---|---|---|---|
| | | | **1.Disruption** | **2.Disruption** | **56°/1000rpm** | | | | **I** | **II** | **III** | **IV** |
| | | | **I-IV** | **only I / II** | **I** | **II** | **III/IV** | | **Mouse tail 10mg** | **Mouse tail 40-55mg** | **Liver 25mg** | **Kidney 18mg** |
| QIAamp Fast | 200µl AVE / 40µl VXL/ 20µl PK / 4µl RNaseA / 1µl DX | Vortex (D) + 5/32" ballcone | 5min | 3min | 20min | 20min | 10min | 265µl MVL | 10,668 | 6,864 | 28,08 | 45,96 |
| | | TissueLyser II +5/32"ballcone | 30sec.24Hz | 15sec.20Hz | | | | | 8,886 | 8,904 | 32,22 | 61,2 |
| | | | 1min 20Hz | 15sec.20Hz | | | - | | 9,048 | 7,5 | - | - |
| | | Vortex (D) + 5/32" ballcone | 5min | 3min | 70min | 70min | 60min | | 7,77 | 7,368 | 26,64 | 40,5 |
| | | TissueLyser II +5/32"ballcone | 30sec.24Hz | 15sec.20Hz | | | | | 8,064 | 8,94 | 30,42 | 60,18 |
| | | | 1min 20Hz | 15sec.20Hz | | | - | | 7,806 | 7,242 | - | - |
| | | Vortex (D) + 5/32" ballcone | 5min | - | - | - | 180min | | - | - | 22,26 | 40,26 |
| | | TissueLyser II +5/32"ballcone | 30sec.24Hz | - | | | | | - | - | 26,7 | 65,4 |
| | | | 1min 20Hz | - | | | - | | - | - | - | - |
| | | Vortex (D) + 5/32" ballcone | 5min | 3min | o/n | - | o/n | | 1,656 | - | 29,76 | 49,98 |
| | | TissueLyser II +5/32"ballcone | 30sec.24Hz | 15sec.20Hz | | | | | 0,84 | - | 18 | 71,4 |
| | | | 1min 20Hz | 15sec.20Hz | | | - | | 0,4548 | - | - | - |
| QIAamp Mini | 180µl ATL + 20µl PK | - | - | - | 60min | | | 200µl AL / 200µl 96-100% EtOH | - | - | 0,23 | 3,10 |
| | | | | | 120min | | | | - | - | 0,333 | - |
| | | | | | 180min | | | | - | - | 0,4416 | 5,42 |
| | | | | | o/n | | | | 3,70 | 9,41 | 0,3852 | 1,76 |

| | | | | | | | | | | | | |
|---|---|---|---|---|---|---|---|---|---|---|---|---|
| Material: Rat liver, RNALater stabilized // Mouse tail, fresh-frozen; Vortex (D): VortexGenie2 +Vertical Microtube Holder(SI-V524) | | | | | | | | | | | | |

**Table 6**

| **Prep** | **Disruption/Lysis Buffer Mix** | **Disruption Device/Particle** | **Mechanical Disruption** | | **Chemical Disruption/Incubation** | | | **Binding** | **Inhibition PCR: QuantiFast Pathogen+ICDNA (12000copies/reaction) [ct value], MV** | | | |
|---|---|---|---|---|---|---|---|---|---|---|---|---|
| | | | 1. Disruption | 2. Disruption | 56°C/1000rpm | | | | Mouse tail 10mg (I) | | Mouse tail 40-55mg (II) | |
| | | | I-IV | only I / II | I | II | III/IV | | 1 µl eluate | 10 µl eluate | 1 µl eluate | 10 µl eluate |
| QIAamp Fast | 200µl AVE / 40µl VXL / 20µl PK / 4µl RNaseA / 1µl DX | Vortex (D) + 5/32" ballcone | 5min | 3min | 20min | 20min | 10min | 265µl MVL | 29,29 | 45 | 29,11 | 45 |
| | | TissueLyser II | 30sec.24Hz | 15sec.20Hz | | | | | 28,195 | 45 | 27,755 | 42,79 |
| | | +5/32"ballcone | 1min 20Hz | 15sec.20Hz | | | - | | 28,6 | 45 | 27,655 | 41,825 |
| | | Vortex (D) + 5/32" ballcone | 5min | 3min | 70min | 70min | 60min | | 27,62 | 42,89 | 27,61 | 30,49 |
| | | TissueLyser II | 30sec.24Hz | 15sec.20Hz | | | | | 28,05 | 36,64 | 27,58 | 32,035 |
| | | +5/32"ballcone | 1min 20Hz | 15sec.20Hz | | | - | | 27,355 | 35,135 | 27,52 | 31,38 |
| | | Vortex (D) + 5/32" ballcone | 5min | - | | | 180min | | - | - | - | - |
| | | TissueLyser II | 30sec.24Hz | - | | | | | | - | - | |
| | | +5/32"ballcone | 1min 20Hz | - | | | - | | - | - | - | - |
| | | Vortex (D) + 5/32" ballcone | 5min | 3min | o/n | - | o/n | | 27,36 | 28,98 | - | - |
| | | TissueLyser II | 30sec.24Hz | 15sec.20Hz | | | | | 27,31 | 30,085 | - | - |
| | | +5/32"ballcone | 1min 20Hz | 15sec.20Hz | | | - | | 27,24 | 28,47 | - | - |
| QIAamp Mini | 180µl ATL + 20µl PK | - | - | - | 60min | | | 200µl AL / 200µl 96-100% EtOH | - | - | - | - |
| | | | | | 120min | | | | - | - | - | - |
| | | | | | 180min | | | | - | - | - | - |
| | | | | | o/n | | | | 40,2 | 45 | 43,62 | 45 |

Additional information table6: IC DNA: ct value = 27,37; ct value =27,85 (MV, n=4)

The new method can achieve higher yields in 10 min disruption + 10 min proteinaseK digestion than are achieved overnight (o/n) (more than 8 hours) with enzymatic digestion alone.

The experiment also shows effectiveness of the new method with very difficult tissue material, such as mouse tail.

### C. Evaluation of the performance with different disrupting particles

The effectiveness of the method of the present invention has been evaluated in view of different disrupting particles, applying the test conditions as given above.

### Test results

**Table 7**

| **Prep** | **Disruption/Lysis Buffer Mix** | **Disruption Device/Particle** | **Disru ption Particle** | | **Mechanical Disruption** | **Chemical Disruption Incubation** | | **Binding** | **DNA Yield [µg], MV n=2** |
|---|---|---|---|---|---|---|---|---|---|
| QIAamp Fast | 200µl AVE / 40µl VXL / 20µl PK / 4µl RNaseA / 1µl DX | Vortex (D) + 5/16" ballcone (full speed) | 1,4mm | Ceramic Beads | 10min | 56°C / 1000rpm | 20min | 265µl MVL | 17,46 |
| | | | 2mm | Ceramic Pearls | 5min | | 10min | 265µl MVL | 20,22 |
| | | | GlassBeads (<1mm) | PathogenLysisTubeL QIAGEN | 10min | | 70min | 265µl MVL | 23,4 |
| | | | 0,8-1mm | Diamond Pearls | 10min | | 70min | 265µl MVL | 17,544 |
| | | | Mesh30/40 | Garnet Sand | 10min | | 20min | 265µl MVL | 11,79 |
| | | | 0,7mm | Garnet Flakes | 5min | | 10min | 265µl MVL | 15,78 |
| | | | 1/8" | Ballcone | 10min | | 20min | 265µl MVL | 29,04 |
| | | | 3x 1/8" | Ballcone | 5min | | 10min | 265µl MVL | 27,84 |
| | | | 3/16" | Ballcone | 5min | | 10min | 265µl MVL | 29,34 |
| | | | 5/32" | Ballcone | 5min | | 10min | 265µl MVL | 27,72 |
| QIAamp Mini | 180µl ATEL + 20µl PK | - | - | - | - | 56°C / 1000rpm | 150min | 200µl AL / 200µl 96-100% EtOH | 0,5754 |

| | | | | | | | | | |
|---|---|---|---|---|---|---|---|---|---|
| Material: Liver, Rat, RNALater stabilized, 15mg; Vortex (D): VortexGenie2 +Vertical Microtube Holder(SI-V524) | | | | | | | | | |

The new chemistry is not restricted to a specific form of disrupting particle.

It has been shown that with the composition of the present invention DNA quality was equally high when using round 5 mm beads or the 5/32" Ballcone.

When using round 5 mm beads, the disruption tended to be decreased and more or larger tissue residues remained compared to the 5/32" Ballcone disruption.

Lysis times were essentially the same, however the disruption with the vortexer achieved slightly lower yields, depending on the used tissue material.

### D. Evaluation of different tissue types

The effectiveness of the method of the present invention has been evaluated in view of different tissue materials, , applying the test conditions as given above.

### Test results

**Table 8**

| **Prep** | **Disruption/Lysis Buffer Mix** | **Disruption Device/Particle** | **Disruption** | **Chemical Disruption/Incubation** | **Binding** | **[µg], MV** n=2 | | | | | | | | | |
|---|---|---|---|---|---|---|---|---|---|---|---|---|---|---|---|
| | | | | **56°C / 1000rpm** | | **Liver, Rat 10mg** | **Brain, Rat 10mg** | **Lung, Rat 10mg** | **Heart, Rat 10mg** | ND **DNA Yield Kidney, Rat 10mg** | **Spleen, Rat 10mg** | **Esophagus, Rat 10mg** | **Trachea, Rat 10mg** | **Ear, Rat 10mg** | **Fat, Rat 20mg** |
| QIAamp Fast | 40µl VXL/ 20µl PK / 4µl RNaseA / 1µl DX | Vortex (D) + 5/32" ballcone | 5min full speed | 10min | 265µl MVL | 13,92 | 4,28 | 11,848 | 5,608 | 15,664 | 63,52 | 13,432 | 23,6 | 21,36 | 1,5328 |
| | 40µl VXL / 20µl PK / 4µl RNaseA / 1µl DX | TissueLyser II + 5/32" ballcone | 30sec.24Hz | 10min | 265µl MVL | 15,528 | 6,808 | 8,944 | 6,968 | 19,76 | 87,52 | 10,352 | 55,92 | 24,24 | 1,6264 |
| | 40µl VXL / PK / 4µl RNaseA / 1µl DX | TissueLyser LT + 5/32" ballcone | 2min 45Hz | 10min | 265µl MVL | 14,104 | 7,392 | 19,76 | 6,544 | 18,88 | 52,8 | 12,144 | 73,68 | 23,92 | 1,5968 |
| QIAamp Mini | 180µl ATL + 20µl PK | - | - | o/n | 200µl AL/ 200µl 96-100% EtOH | 2,39 | 0,35 | 1,54 | 0,88 | 5,30 | 7,22 | 1,23 | 1,33 | 4,06 | 0,78 |

| | | | | | | | | | | | | | | | |
|---|---|---|---|---|---|---|---|---|---|---|---|---|---|---|---|
| Material: fresh-frozen; Vortex (D); VortexGenie2 +Vertical Microtube Holder(SI-V524) | | | | | | | | | | | | | | | |

**Table 9**

| **Prep** | **Disruption / Lysis Buffer Mix** | **Disruption Device/ Particle** | **Mechanical Disruption** | **Chemical Disruption / Incubation** | **Binding** | **DNA Yield [µg], MV n=2** | | |
|---|---|---|---|---|---|---|---|---|
| | | | | **56°C/ 1000rpm** | | **I=Tail(tip), Rat 0,5cm n=1** | **II=Tail, Mouse 1cmx0,2cm n=1** | **III=Ear, Pig MV, 10mg** n=2 |
| QIAamp Fast | 40µl VXL/20µl PK/ 4µl RNaseA /1µl DX | Vortex (D) + 5/32" ballcone | 2x 5min full speed | 1=70min | 265^ MVL | 28 | 7,696 | 10,28 |
| | | TissueLyser II + 5/32" ballcone | 2x 30sec.24Hz | II=50min | 265µl MVL | 38,08 | 14,752 | 10,392 |
| | | TissueLyser LT + 5/32" ballcone | 2x 2min 45Hz | III=20min | 265µl MVL | 28,16 | 14,448 | 10,856 |
| QIAamp Mini | 180µl ATL + 20µl PK | | | 56°C/ 1000rpm/ o/n | 200µl AL/ 200µl 96-100% EtOH | 8,40 | 10,22 | 0,84 |
| Material: fresh-frozen; Vort VortexGenie2 + Vertical Microtube Holder (SI-V524) | | | | | | | | |

The improvements as described herein can be achieved with different kinds of tissue samples, such as soft tissue materials and even with tough tissue materials such as mouse tail.

### E. Evaluation of synergistic effects

The effectiveness of the combination of mechanical and chemical (enzymatic) lysis compared to the individual treatments has been evaluated, applying the test conditions as given above.

### Test results:

**Table 10**

| **Prep** | **Disruption Device/ Particle** | **Disruption Buffer** | **Chemical disruption incubation** | **visual assessment of homogenization** | | **Binding Buffer** | **Yield** | |
|---|---|---|---|---|---|---|---|---|
| | | | | **Liver** | **Muscle** | | **Liver 10 mg** | **Muscle 10 mg** |
| Combined chemical and mechanical disruption | Vortex (D) + 5/16" ballcone | 200µl AVE/ 40µl VXL /20µl PK /4µl RNaseA /1µl DX | 56°C/1000rpm/ 10min | ok | ok | 265µl MVL | 25,14 | 3,49 |
| Mechanical disruption only | Vortex (D) + 5/16" ballcone | 220µl AVE/ 40µl VXL /4µl RNaseA /1µl DX | - | ok | tiny pieces of tissue left | 265µl MVL | 5,45 | 0,75 |
| Chemical disruption only | - | 200µl AVE/ 40µl VXL /20µl PK /4µl RNaseA | 56°C/1000rpm/ 150min | tiny pieces of tissue left | ok | 265µl MVL | 1,05 | 0,9 |
| | | | | | | | MV, n=2 | |

Yields are in micrograms, as measured by a DN- specific flourescent assay (Qubit 2.0) A synergistic effect (x-fold improvement over sum of both individual methods) can be seen:

| Liver 10 mg | Muscle 10 mg |
|---|---|
| 3,87 | 2,12 |

### F. Evaluation of improvement versus commercially available test kits

The effectiveness of the composition of the present invention compared to several commercially available test kits has been evaluated.

### Test results:

**Table 11**

| **Company** | **Pre Kit** | **Disruption tube / Disruption Particles** | **Disruption Device** | **Chemical Disruption / Incubation** | | **DNA Yield [µg], MV n=2** | | |
|---|---|---|---|---|---|---|---|---|
| | | | | | | **RNALater 10mg** | | |
| | | | | | | **I** | **II** | **III** |
| | | | | | | **Kidney, Rat** | **Liver, Rat** | **Lung, Rat** |
| **Zymo** | ZR Genomic DNA-Tissue Mini Prep | - | - | PK-lysis | 55°C/o/n | 3,365 | 10,685 | 5,85 |
| | Quick-gDNA Mini Prep | - | Squisher | - | - | 1,92 | 1,655 | 1,575 |
| MPBio | FastDNA Green Spin Kit - Prep1 | LysingMatrixD (2ml Screwcap tube with 1.4mm ceramic sphere) +6mm ceramic sphere | Fast Prep | - | - | 4,46 | 1,9 | 3,775 |
| | FastDNA Green Spin Kit - Prep2 (Rep.) | | Fast Prep | - | - | 2,0345 | 0,9555 | 0,8155 |
| Invitrogen | PureLink Genomic DNA Mini Kit | - | - | PK-lysis | 55°C / vortex now and again / 240min | 1,715 | 0,644 | 2,89 |
| MoBio | UltraClean Tisue&Cells DNA Isolation | TD1 solution (GuHCl, Isopropanol) Dry Bead tube (2ml Screwcap tube with 0.7 mm Garnet Flakes) | vortex | - | - | 16,2 | 6,615 | 9,565 |
| | | | vortex | PK-lysis | 60°C /30min | 11,45 | 3,615 | 14,9 |
| | | - | **-** | PK-lysis | 56°C / 1000rpm / 180min | 2,705 | 0,4025 | 2,285 |
| QIAGEN | QIAamp Mini | - | - | PK-lysis | 56°C / 1000rpm / o/n | 1,315 | 0,4485 | 1,98 |
| | | no disrupting particles | TissueRuptor (mixer) | PK-lysis | 56°C / 1000rpm / 120min | 5,885 | 7,585 | 10,05 |
| QIAGEN | QIAamp Fast | Tissue Disruption Tube (2ml Screwcap tube with skirted base and 5/32" ballcone) | vortex (D) | PK-lysis | 56°C / 1000rpm / 10min (I,II) / 20min (III) | 35,9 | 23 | 52,95 |
| | | | TissueLyser II | PK-lysis | 56°C / 1000rpm / 10min (I,II) / 20min (III) | 27,95 | 24,5 | 59 |
| | | | TissueLyser LT | PK-lysis | 56°C / 1000rpm / 10min (I,II) / 20min (III) | 42,6 | 29,2 | 33,6 |

**Table 12**

| **Company** | **Prep Kit** | **Disruption tube/ Disruption Particles** | **Disruption Device** | **Chemical Disruption/Incubation** | | **Prep Time [min]** | | |
|---|---|---|---|---|---|---|---|---|
| | | | | | | **RNALater 10mg** | | |
| | | | | | | **I Kidney, Rat** | **II Liver, Rat** | **III Lung, Rat** |
| Zymo | ZR Genomic DNA-Tissue Mini Prep | | - | PK-lysis | 55°C/o/n | 1200 | 1200 | 1200 |
| | Quick-gDNA Mini Prep | - | Squisher | - | - | 25 | 25 | 25 |
| MPBio | FastDNA Green Spin Kit - Prep1 | LysingMatrixD (2ml Screwcap tube with | Fast Prep | - | - | 100 | 100 | 100 |
| | FastDNA Green Spin Kit - Prep2 (Rep.) | 1.4mm ceramic sphere) +6mm ceramic sphere | Fast Prep | - | - | 110 | 110 | 110 |
| Invitrogen | PureLink Genomic DNA Mini Kit | - | - | PK-lysis | 55°C/vortex now and again/240min | 265 | 265 | 265 |
| MoBio | UltraClean Tisue&Cells DNA Isolation | TD1 solution (GuHCl, | vortex | - | - | 30 | 30 | 30 |
| | | Isopropanol) Dry Bead tube (2ml Screwcap tube with 0.7 mm Garnet Flakes) | vortex | PK-lysis | 60°C/30min | 60 | 60 | 60 |
| QIAGEN | QIAamp Mini | - | - | PK-lysis | 56°C/1000rpm/ 180min | 200 | 200 | 200 |
| | | - | - | PK-lysis | 56°C/1000rpm/o/n | 1200 | 1200 | 1200 |
| | | no disrupting particles | TissueRuptor (mixer) | PK-lysis | 56°C/1000rpm/ 120min | 150 | 150 | 150 |
| QIAGEN | QIAamp Fast | Tissue Disruption Tube (2ml Screwcap tube with skirted base and 5/32" | vortex (D) | PK-lysis | 56°C/1000rpm/10min (I,II)/20min (III) | 35 | 35 | 45 |
| | | | TissueLyser II | PK-lysis | 56°C/1000rpm/10min (I,II)/20min (III) | 30 | 30 | 40 |
| | | | ballcone) TissueLyser LT | PK-lysis | 56°C/1000rpm/10min (I,II)/20min (III) | 35 | 35 | 45 |

**Table 13**

| **Company** | **Prep Kit** | **Disruption tube / Disruption Particles** | **Disruption Device** | **Chemical disruption/Incubation** | | **DNA Yield [µg], n=1** | **Prep Time [min] =** |
|---|---|---|---|---|---|---|---|
| | | | | | | **IV** | |
| | | | | | | **Mousetail, fresh-f IV Mousetail, fresh frozen, 1cm** | |
| Zymo | ZR Genomic DNA-Tissue Mini Prep | - | - | PK-lysis | 55°C/o/n | 17 | 1200 |
| | Quick-gDNA Mini Prep | - | Squisher | - | - | 4,58 | 25 |
| MPBio | FastDNA Green Spin Kit - Prep1 | LysingMatrixD (2ml Screwcap tube with 1.4mm | Fast Prep | - | - | 0,367 | 100 |
| | FastDNA Green Spin Kit - Prep2 (Rep.) | ceramic sphere) +6mm ceramic sphere | Fast Prep | - | - | 0,262 | 120 |
| Invitrogen | PureLink Genomic DNA Mini Kit | - | - | PK-lysis | 55°C/vortex now and again/o/n | 5,1 | 265 |
| MoBio | UltraClean Tisue&Cells DNA Isolation | TD1 solution (GuHCl, | vortex | - | - | 0 | 30 |
| | | Isopropanol) Dry Bead tube (2ml Screwcap tube with 0.7 mm Garnet Flakes) | vortex | PK-lysis | 60°C/30min | 0,109 | 60 |
| QIAGEN | QIAamp Fast | Tissue Disruption Tube (2ml Screwcap tube with skirted base and 5/32" ballcone) | vortex (D) | PK-lysis | 56°C/1000rpm/ 20min (IV) | 10,4 | 45 |
| | | | TissueLyser II | PK-lysis | 56°C/1000rpm/ 10min (IV) | 20,5 | 40 |
| | | | TissueLyser LT | PK-lysis | 56°C/1000rpm/ 10min (IV) | 11,8 | 45 |

| | | | | | | | |
|---|---|---|---|---|---|---|---|
| Vortex (D): VortexGenie2 +Vertical Microtube Holder(SI-V524) | | | | | | | |

The composition of the present invention achieves improved yields with minimum to no DNA degradation (improved DNA quality) with improved time effort compared to commercially available kits.

**Table 12 (continued)**

| **Company** | **Prep Kit** | **Disruption tube / Disruption Particles** | **Disruption Device** | **Chemic In Disruption / Incubation** | | **DNA Yield [µg], MV n=2** | | |
|---|---|---|---|---|---|---|---|---|
| | | | | | | **RNALater 10mg** | | |
| | | | | | | **I** | **II** | **III** |
| | | | | | | **Kidney, Rat** | **Liver, Rat** | **Lung, Rat** |
| QIAGEN | QIAamp Mini | - | - | PK-lysis | 56°C / 1000rpm / 180min | 2,705 | 0,4025 | 2,285 |
| | | - | - | PK-lysis | 56°C / 1000rpm / o/n | 1,315 | 0,4485 | 1,98 |
| | | no disrupting particles | TissueRuptor (mixer) | pK-lysis | 56°C / 1000rpm / 120min | 5,885 | 7,585 | 10,05 |
| QIAGEN | QIAamp Fast | Tissue Disruption Tube (2ml Screwcap tube with skirted base and 5/32" ballcone) | vortex (D) | PK-lysis | 56°C / 1000rpm / 10min (I,II) / 20min (III) | 35,9 | 23 | 52,95 |
| | | | TissueLyser II | PK-lysis | 56°C / 1000rpm / 10min (I,II) / 20min (III) | 27,95 | 24,5 | 59 |
| | | | TissueLyser LT | PK-lysis | 56°C / 1000rpm / 10min (I,II) / 20min (III) | 42,6 | 29,2 | 33,6 |

**Table 13**

| **Company** | **Prep Kit** | **Disruption tube / Disruption Particles** | **Disruption Device** | **Chemical Disruption / Incubation** | | **Prep Time [min]** | | |
|---|---|---|---|---|---|---|---|---|
| | | | | | | **RNALater 10mg** | | |
| | | | | | | **I** | **II** | **III** |
| | | | | | | **Kidney, Rat** | **Liver, Rat** | **Lung, Rat** |
| Zymo | ZR Genomic DNA-Tissue Mini Prep | - | - | PK-lysis | 55°C/ o/n | 1200 | 1200 | 1200 |
| | Quick-gDNA Mini Prep | - | Squisher | - | - | 25 | 25 | 25 |
| MPBio | FastDNA Green Spin Kit - Prep1 | LysingMatrixD (2ml Screwcap tube with | Fast Prep | - | - | 100 | 100 | 100 |
| | FastDNA Green Spin Kit - Prep2 (Rep.) | 1.4mm ceramic sphere) +6mm ceramic sphere | Fast Prep | - | - | 110 | 110 | 110 |
| Invitrogen | PureLink Genomic DNA Mini Kit | - | - | PK-lysis | 55°C / vortex now and again / 240min | 265 | 265 | 265 |
| MoBio | UltraClean Tisue&Cells DNA Isolation | TD1 solution (GuHCl, | vortex | - | - | 30 | 30 | 30 |
| | | Isopropanol) Dry Bead tube (2ml Screwcap tube with 0.7 mm Garnet Flakes) | vortex | PK-lysis | 60°C / 30min | 60 | 60 | 60 |
| QIAGEN | QIAamp Mini | - | - | PK-lysis | 56°C / 1000rpm / 180min | 200 | 200 | 200 |
| | | - | - | PK-lysis | 56°C / 1000rpm /o/n | 1200 | 1200 | 1200 |
| | | no disrupting particles | TissueRuptor (mixer) | PK-lysis | 56°C / 1000rpm / 120min | 150 | 150 | 150 |
| QIAGEN | QIAamp Fast | Tissue Disruption Tube (2ml Screwcap tube with skirted base and 5/32" ballcone) | vortex (D) | PK-lysis | 56°C / 1000rpm / 10min (I,II) / 20min (III) | 35 | 35 | 45 |
| | | | TissueLyser II | PK-lysis | 56°C / 1000rpm / 10min (I,II) / 20min (III) | 30 | 30 | 40 |
| | | | TissueLyser LT | PK-lysis | 56°C / 1000rpm / 10min (I,II) / 20min (III) | 35 | 35 | 45 |

**Table 14**

| **Company** | **Prep Kit** | **Disruption tube / Disruption Particles** | **Disruption Device** | **Chemical disruption / Incubation** | | **DNA Yield [µg], n=1** | **Prep Time [min]** |
|---|---|---|---|---|---|---|---|
| | | | | | | **IV Mousetail, fresh-frozen, 1cm** | |
| Zymo | ZR Genomic DNA-Tissue Mini Prep | - | - | PK-lysis | 55°C/o/n | 17 | 1200 |
| | Quick-gDNA Mini Prep | - | Squisher | - | - | 4,58 | 25 |
| MPBio | FastDNA Green Spin Kit - Prep1 | LysingMatrixD (2ml Screwcap tube with 1.4mm ceramic sphere) +6mm ceramic sphere | Fast Prep | - | - | 0,367 | 100 |
| | FastDNA Green Spin Kit - Prep2 (Rep.) | | Fast Prep | - | - | 0,262 | 120 |
| Invitrogen | PureLink Genomic DNA Mini Kit | - | - | PK-lysis | 55°C/ vortex now and again/o/n | 5,1 | 265 |
| MoBio | UltraClean Tisue&Cells DNA Isolation | TD1 solution (GuHCl, Isopropanol) Dry Bead tube (2ml Screwcap tube with 0.7 mm Garnet Flakes) | vortex | - | - | 0 | 30 |
| | | | vortex | PK-lysis | 60°C / 30min | 0,109 | 60 |
| QIAGEN | QIAamp Fast | Tissue Disruption Tube (2ml Screwcap tube with skirted base and 5/32" ballcone) | vortex (D) | PK-lysis | 56°C / 1000rpm / 20min (IV) | 10,4 | 45 |
| | | | TissueLyser II | PK-lysis | 56°C / 1000rpm / 10min (IV) | 20,5 | 40 |
| | | | TissueLyser LT | PK-lysis | 56°C / 1000rpm / 10min (IV) | 11,8 | 45 |

| | | | | | | | |
|---|---|---|---|---|---|---|---|
| Vortex (D): VortexGenie2 +Vertical Microtube Holder(SI-V524) | | | | | | | |

The composition of the present invention achieves improved yields with minimum to no DNA degradation (improved DNA quality) with improved time effort compared to commercially available kits.

## Claims

1. Use of a composition comprising
- one or more solid disrupting particles;
- at least one enzyme for enzymatic lysis;
- at least one chaotropic agent in a total concentration equal to or less than 1M; and preferably
- at least one buffer
for disrupting human and animal derived solid tissue material.

2. The use according to claim 1, wherein the composition further comprises one or more agents selected from
- at least one surfactant, preferably selected from the group of non-ionic surfactants;
- at least one nuclease inhibitor;
- at least one anti-foaming agent;
- at least one osmotic stabilizer;
- at least one reducing agent;
and mixtures thereof.

3. The use according to any one of the preceding claims, wherein the composition further comprises at least one additional enzyme, other than the enzyme for enzymatic lysis, preferably an RNase.

4. The use according to any one of the preceding claims, wherein the composition comprises at least one enzyme for enzymatic lysis, which is selected from the group of proteases, preferably proteinaseK.

5. The use according to any one of the preceding claims, wherein the human and animal derived solid tissue is **characterized by** comprising cellular organizational level intermediates between cells and a complete organ, a fibrous and / or membranous structure, and is preferably selected from connective tissue, muscle tissue, nervous tissue, epithelial tissue and mineralized tissue, such as preferably from skin, muscle, tendons, filaments, nerves, cartilage, bone, tail, as well as organs, such as intestine, gastric, liver, spleen, brain, lymph, bone marrow, kidney, heart.

6. The use according to any one of the preceding claims, wherein the composition comprises solid disrupting particles having a size of at least 1 mm, preferably of > 2 mm, more preferably of ≥ 3 mm.

7. A composition for disrupting human and animal derived solid tissue material, comprising
- one or more solid disrupting particles;
- at least one enzyme for enzymatic lysis;
- guanidinium hydrochloride in a concentration equal to or less than 1M; and preferably
- at least one buffer.

8. The composition according to claim 7, further comprising one or more agents selected from
- at least one surfactant, preferably selected from the group of non-ionic surfactants;
- at least one nuclease inhibitor;
- at least one anti-foaming agent;
- at least one osmotic stabilizer;
- at least one reducing agent;
- at least one additional enzyme, other than the enzyme for enzymatic lysis, preferably an RNase
and mixtures thereof.

9. A kit for disrupting human and animal derived solid tissue material, the kit comprising
- one or more solid disrupting particles;
- at least one enzyme for enzymatic lysis;
- at least one buffer;
- guanidinium hydrochloride in a concentration equal to or less than 1M;
and optionally one or more agents selected from
- at least one surfactant;
- at least one nuclease inhibitor;
- at least one anti-foaming agent;
- at least one osmotic stabilizer;
- at least one reducing agent;
- at least one additional enzyme, other than the enzyme for enzymatic lysis, preferably an RNase;
- at least one organic solvent, preferably an alcohol; and
- optionally a container for receiving the tissue material;
- and a leaflet with instructions for processing the tissue material.

10. A combination comprising the composition as defined in claim 7 or 8 or the kit as defined in claim 9 and a device for effecting milling of the tissue material, preferably a high-power mixing mill, a high-speed mixer, or a low-power mixer, including a vortexer and optionally further comprising a human and animal derived solid tissue material for disruption.

11. A method for disrupting human and animal derived solid tissue material, wherein the solid tissue material is subjected to a simultaneous treatment of
- mechanical disruption by grinding, milling or beating and
- chemical disruption by enzymatic lysis,
by grinding, milling or beating the tissue material in a lysis solution which comprises one or more solid disrupting particles, at least one enzyme for enzymatic lysis, and at least one chaotropic agent in a total concentration equal to or less than 1M.

12. The method according to claim 11, wherein the at least one chaotropic agent is guanidinium hydrochloride.

13. The method according to claim 11 or 12, wherein disruption is carried out by using low-power mixers including vortexers, preferably by using vortexers.

14. The method according to any one of claims 11 to 13, which is carried out by using the composition as defined in claim 7 or 8, the kit as defined in claim 9 or the combination as defined in claim 10.

15. The method according to any one of claims 11 to 14, comprising the steps:
(i) adding to a human and animal derived solid tissue material in a suitable container
a) the one or more solid disrupting particles, the at least one buffer, the at least one enzyme for enzymatic lysis, the at least one chaotropic agent in a total concentration equal to or less than 1M, optionally an RNase, and optionally one or more agents selected from surfactants, osmotic stabilizers, reducing agents, nuclease inhibitors, anti-foaming agents or mixtures thereof, or
b) the composition as defined in claim 7 or 8;
(ii) optionally closing the container;
(iii) disrupting the solid tissue material in the container using a high-power mixing mill, a high-speed mixer, or a low-power mixer, including a vortexer, preferably by using a vortexer;
(iv) incubating the mixture of step (iv) at a temperature above 24 °C;
(v) optionally repeating step (iii) and (iv);
(vi) optionally providing the resulting mixture for subsequent purification, separation, extraction and/or analytic process steps.

## Patentansprüche

1. Verwendung einer Zusammensetzung, umfassend
- ein oder mehrere feststoffaufbrechende Partikel;
- mindestens ein Enzym zur enzymatischen Lyse;
- mindestens ein chaotropes Mittel in einer Gesamtkonzentration gleich oder kleiner als 1M; und bevorzugt
- mindestens einen Puffer
zum Aufbrechen von von Mensch und Tier stammendem festen Gewebematerial.

2. Verwendung nach Anspruch 1, wobei die Zusammensetzung weiter ein oder mehrere Mittel, ausgewählt aus
- mindestens einem Tensid, bevorzugt ausgewählt aus der Gruppe der nichtionischen Tenside;
- mindestens einem Nuklease-Inhibitor:
- mindestens einem Antischaummittel;
- mindestens einem osmotischen Stabilisator;
- mindestens einem Reduktionsmittel;
und Mischungen davon umfasst.

3. Verwendung nach einem der vorstehenden Ansprüche, wobei die Zusammensetzung weiter mindestens ein zusätzliches Enzym, das kein Enzym für enzymatische Lyse ist, bevorzugt eine RNase, umfasst.

4. Verwendung nach einem der vorstehenden Ansprüche, **dadurch gekennzeichnet, dass** die Zusammensetzung mindestens ein Enzym zur enzymatischen Lyse enthält, das ausgewählt ist aus der Gruppe der Proteasen, bevorzugt ProteinaseK.

5. Verwendung nach einem der vorstehenden Ansprüche, wobei das von Mensch und Tier stammende feste Gewebe **dadurch gekennzeichnet ist, dass** es zelluläre Organisationsstufen zwischen Zellen und einem vollständigen Organ, eine faserige und/oder membranöse Struktur umfasst und bevorzugt ausgewählt ist aus Bindegewebe, Muskelgewebe, Nervengewebe, Epithelgewebe und mineralisiertem Gewebe, wie bevorzugt aus Haut, Muskel, Sehnen, Filamenten, Nerven, Knorpel, Knochen, Schwanz, sowie Organen, wie Darm, Magen, Leber, Milz, Gehirn, Lymphe, Knochenmark, Niere, Herz.

6. Verwendung nach einem der vorstehenden Ansprüche, wobei die Zusammensetzung Feststoff-aufbrechende Partikel umfasst, die eine Größe von mindestens 1 mm, bevorzugt von > 2 mm, besonders bevorzugt von ≥ 3 mm aufweisen.

7. Zusammensetzung zum Aufbrechen von von Mensch und Tier stammendem festen Gewebematerial, umfassend
- ein oder mehrere Feststoff-aufbrechende Partikel;
- mindestens ein Enzym zur enzymatischen Lyse;
- Guanidiniumhydrochlorid in einer Konzentration gleich oder kleiner als 1M; und bevorzugt
- mindestens einen Puffer.

8. Zusammensetzung nach Anspruch 7, weiter umfassend ein oder mehrere Mittel, ausgewählt aus
- mindestens einem Tensid, bevorzugt ausgewählt aus der Gruppe der nichtionischen Tenside;
- mindestens einem Nuklease-Inhibitor:
- mindestens einem Antischaummittel;
- mindestens einem osmotischen Stabilisator;
- mindestens einem Reduktionsmittel;
- mindestens einem zusätzliches Enzym, das kein Enzym für enzymatische Lyse ist, bevorzugt eine RNase
und Mischungen davon.

9. Kit zum Aufbrechen von von Mensch und Tier stammendem festen Gewebematerial, das Kit umfassend
- ein oder mehrere Feststoff-aufbrechende Partikel;
- mindestens ein Enzym zur enzymatischen Lyse;
- mindestens einen Puffer;
- Guanidiniumhydrochlorid in einer Konzentration gleich oder kleiner als 1M; und optional ein oder mehrere Mittel, ausgewählt aus
- mindestens einem Tensid;
- mindestens einem Nuklease-Inhibitor:
- mindestens einem Antischaummittel;
- mindestens einem osmotischen Stabilisator;
- mindestens einem Reduktionsmittel;
- mindestens einem zusätzliches Enzym, das kein Enzym für enzymatische Lyse ist, bevorzugt eine RNase;
- mindestens einem organischen Lösungsmittel, bevorzugt einem Alkohol; und
- optional ein Gefäß zur Aufnahme des Gewebematerials;
- und einen Beipackzettel mit Anweisungen zur Verarbeitung des Gewebematerials.

10. Kombination, umfassend die Zusammensetzung nach Anspruch 7 oder 8 oder das Kit nach Anspruch 9 und eine Vorrichtung zum Bewirken des Zerkleinerns des Gewebematerials, bevorzugt ein Hochleistungs-Mischwalzwerk, ein Hochgeschwindigkeitsmischer oder ein Niederleistungsmischer, einschließlich eines Vortexers, und optional weiter umfassend ein von Mensch und Tier stammendes festes Gewebematerial zum Aufbrechen.

11. Verfahren zum Aufbrechen von von Mensch und Tier stammendem festen Gewebematerial, wobei das feste Gewebematerial einer gleichzeitigen Behandlung
- des mechanischen Aufbrechens durch Zerkleinern, Mahlen oder Schlagen und
- des chemischen Aufbrechens durch enzymatische Lyse unterzogen wird,
durch Zerkleinern, Mahlen oder Schlagen des Gewebematerials in einer Lyselösung, die ein oder mehrere Feststoff-aufbrechende Partikel, mindestens ein Enzym für die enzymatische Lyse und mindestens ein chaotropes Mittel in einer Gesamtkonzentration gleich oder kleiner als 1M umfasst.

12. Verfahren nach Anspruch 11, wobei das mindestens eine chaotrope Mittel Guanidiniumhydrochlorid ist.

13. Verfahren nach Anspruch 11 oder 12, wobei das Aufbrechen unter Verwendung von Niederleistungsmischern einschließlich Vortexern, bevorzugt unter Verwendung von Vortexern, durchgeführt wird.

14. Verfahren nach einem der Ansprüche 11 bis 13, das unter Verwendung der Zusammensetzung nach Anspruch 7 oder 8, des Kits nach Anspruch 9 oder der Kombination nach Anspruch 10 durchgeführt wird.

15. Verfahren nach einem der Ansprüche 11 bis 14, umfassend die Schritte:
(i) Zugabe zu einem von Mensch und Tier stammenden festen Gewebematerial in einem geeigneten Gefäß
a) das eine oder die mehreren Feststoff-aufbrechenden Partikel, den mindestens einen Puffer, das mindestens eine Enzym zur enzymatischen Lyse, das mindestens eine chaotrope Mittel in einer Gesamtkonzentration gleich oder kleiner als 1M, optional eine RNase und optional ein oder mehrere Mittel, ausgewählt aus Tensiden, osmotischen Stabilisatoren, Reduktionsmitteln, Nuklease-Inhibitoren, Antischaummitteln oder Mischungen davon, oder
b) die Zusammensetzung nach Anspruch 7 oder 8;
(ii) optional Schließen des Gefäßes;
(iii) Aufbrechen des festen Gewebematerials im Gefäß unter Verwendung eines Hochleistungs-Mischwalzwerks, eines Hochgeschwindigkeitsmischers oder eines Niederleistungsmischers, einschließlich eines Vortexers, bevorzugt unter Verwendung eines Vortexers;
(iv) Inkubieren der Mischung aus Schritt (iv) bei einer Temperatur über 24 °C;
(v) optional Wiederholen von Schritt (iii) und (iv);
(vi) optional Bereitstellen der resultierenden Mischung für nachfolgende Reinigungs-, Trenn-, Extraktions- und/oder analytische Verfahrensschritte.

## Revendications

1. Utilisation d'une composition comprenant
- une ou plusieurs particules de désintégration solide ;
- au moins une enzyme destinée à la lyse enzymatique ;
- au moins un agent chaotropique dans une concentration totale inférieure ou égale à 1 M ; et de préférence
- au moins un tampon
pour désintégrer du matériel tissulaire solide d'origine humaine et animale.

2. Utilisation selon la revendication 1, dans laquelle la composition comprend en outre un ou plusieurs agents choisis parmi
- au moins un tensioactif, de préférence choisi parmi le groupe de tensioactifs non ioniques ;
- au moins un inhibiteur de nucléases ;
- au moins un agent antimoussant ;
- au moins un stabilisateur osmotique ;
- au moins un agent réducteur ;
et des mélanges de ceux-ci.

3. Utilisation selon l'une quelconque des revendications précédentes, dans laquelle la composition comprend en outre au moins une enzyme supplémentaire, différente de l'enzyme destinée à la lyse enzymatique, de préférence une RNase.

4. Utilisation selon l'une quelconque des revendications précédentes, dans laquelle la composition comprend au moins une enzyme destinée à la lyse enzymatique, qui est choisie parmi le groupe de protéases, de préférence une protéinase K.

5. Utilisation selon l'une quelconque des revendications précédentes, dans laquelle le tissu solide d'origine humaine ou animale est **caractérisé en ce qu'**il comprend des intermédiaires de niveau d'organisation cellulaire entre les cellules et un organe entier, une structure fibreuse et/ou membraneuse, et est de préférence choisi parmi du tissu conjonctif, du tissu musculaire, du tissu nerveux, du tissu épithélial et du tissu minéralisé, tel que de préférence provenant de la peau, des muscles, des tendons, des filaments, des nerfs, du cartilage, des os, du coccyx, ainsi que des organes, tels que les intestins, l'estomac, le foie, la rate, le cerveau, la lymphe, la moelle osseuse, les reins, le cœur.

6. Utilisation selon l'une quelconque des revendications précédentes, dans laquelle la composition comprend des particules de désintégration solide présentant une taille d'au moins 1 mm, de préférence > 2 mm, de manière davantage préférée ≥ 3 mm.

7. Composition destinée à la désintégration de matériel tissulaire solide d'origine humaine et animale comprenant
- une ou plusieurs particules de désintégration solide ;
- au moins une enzyme destinée à la lyse enzymatique ;
- du chlorhydrate de guanidine dans une concentration inférieure ou égale à 1 M ; et de préférence
- au moins un tampon.

8. Composition selon la revendication 7, comprenant en outre un ou plusieurs agents choisis parmi
- au moins un tensioactif, de préférence choisi parmi le groupe de tensioactifs non ioniques ;
- au moins un inhibiteur de nucléases ;
- au moins un agent antimoussant ;
- au moins un stabilisateur osmotique ;
- au moins un agent réducteur ;
- au moins une enzyme supplémentaire, différente de l'enzyme destinée à la lyse enzymatique, de préférence une RNase
et des mélanges de ceux-ci.

9. Kit de désintégration de matériel tissulaire solide d'origine humaine et animale, le kit comprenant
- une ou plusieurs particules de désintégration solide ;
- au moins une enzyme destinée à la lyse enzymatique ;
- au moins un tampon ;
- du chlorhydrate de guanidine dans une concentration inférieure ou égale à 1M;
et facultativement un ou plusieurs agents choisis parmi
- au moins un tensioactif,
- au moins un inhibiteur de nucléases ;
- au moins un agent antimoussant ;
- au moins un stabilisateur osmotique ;
- au moins un agent réducteur ;
- au moins une enzyme supplémentaire, différente de l'enzyme destinée à la lyse enzymatique, de préférence une RNase ;
- au moins un solvant organique, de préférence un alcool ; et
- facultativement un récipient destiné à recevoir le matériel tissulaire ;
- et une notice mentionnant les instructions à suivre pour le traitement du matériel tissulaire.

10. Combinaison comprenant la composition telle que définie selon la revendication 7 ou 8 ou kit tel que défini selon la revendication 9 et un dispositif destiné à effectuer le broyage du matériel tissulaire, de préférence un malaxeur à haute puissance, un mélangeur à grande vitesse ou un mélangeur à faible puissance, incluant un agitateur-mélangeur à vortex et facultativement comprenant en outre un matériel tissulaire solide d'origine humaine et animale destiné à la désintégration.

11. Procédé de désintégration de matériel tissulaire solide d'origine humaine et animale, dans lequel le matériel tissulaire solide est soumis à un traitement simultané de
- désintégration mécanique par mouture, broyage ou battage et
- désintégration chimique par lyse enzymatique,
par mouture, broyage ou battage du matériel tissulaire dans une solution de lyse qui comprend une ou plusieurs particules de désintégration solide, au moins une enzyme destinée à la lyse enzymatique et au moins un agent chaotropique dans une concentration totale inférieure ou égale à 1 M.

12. Procédé selon la revendication 11, dans lequel l'au moins un agent chaotropique est le chlorhydrate de guanidine.

13. Procédé selon la revendication 11 ou 12, dans lequel la désintégration est réalisée en utilisant des mélangeurs à faible puissance incluant des agitateurs-mélangeurs à vortex, de préférence en utilisant des agitateurs-mélangeurs à vortex.

14. Procédé selon l'une quelconque des revendications 11 à 13, qui est réalisé en utilisant la composition telle que définie selon la revendication 7 ou 8, le kit tel que défini selon la revendication 9 ou la combinaison telle que définie selon la revendication 10.

15. Procédé selon l'une quelconque des revendications 11 à 14, comprenant les étapes consistant à :
(i) ajouter à un matériel tissulaire solide d'origine humaine et animale dans un récipient approprié
(a) l'une ou plusieurs des particules de désintégration solide, l'au moins un tampon, l'au moins une enzyme destinée à la lyse enzymatique, l'au moins un agent chaotropique dans une concentration totale inférieure ou égale à 1 M, facultativement une RNase, et facultativement un ou plusieurs agents choisis parmi des tensioactifs, stabilisateurs osmotiques, agents réducteurs, inhibiteurs de nucléases, agents antimoussants ou des mélanges de ceux-ci, ou
(b) la composition telle que définie selon la revendication 7 ou 8 ;
(ii) facultativement fermer le récipient ;
(iii) désintégrer le matériel tissulaire solide dans le récipient en utilisant un malaxeur à haute puissance, un mélangeur à grande vitesse ou un mélangeur à faible puissance, incluant un agitateur-mélangeur à vortex, de préférence en utilisant un agitateur-mélangeur à vortex ;
(iv) incuber le mélange de l'étape (iv) à une température supérieure à 24 °C ;
(v) facultativement répéter les étapes (iii) et (iv) ;
(vi) facultativement fournir le mélange obtenu pour réaliser les étapes ultérieures de purification, séparation, extraction et/ou traitement analytique.
